# EUROPEAN PATENT SPECIFICATION

(11) **EP 3 492 606 B1**
(45) Date of publication and mention of the grant of the patent: **04.10.2023**
(21) Application number: 18198254.7
(22) Date of filing: 22.08.2012
(51) Int. Cl.: C12Q 1/6886

(54) **URINE BIOMARKERS**
URINBIOMARKER
BIOMARQUEURS URINAIRES

(30) Priority: 22.08.2011 US 201161526238 P; 17.11.2011 US 201161561092 P; 09.04.2012 US 201261621693 P
(43) Date of publication of application: 05.06.2019
(62) Divisional of application: 12826005.6
(73) Proprietor: Exosome Diagnostics, Inc., Waltham, MA 02451 (US)
(72) Inventor: RUSSO, Leileata M., Belmont, MA 02478 (US)
(74) Representative: Cooley (UK) LLP

(56) References cited:
- WO-A1-2009/126122
- WO-A2-2005/113816
- WO-A2-2010/062706
- NILSSON J ET AL: "Prostate cancer-derived urine exosomes: a novel approach to biomarkers for prostate cancer", BRITISH JOURNAL OF CA, NATURE PUBLISHING GROUP, GB, vol. 100, no. 10, 1 May 2009 (2009-05-01), pages 1603-1607, XP008149487, ISSN: 0007-0920, DOI: 10.1038/SJ.BJC.6605058 [retrieved on 2009-04-28]

## Description

### FIELD OF INVENTION

The present invention relates generally to the field of urine biomarker analysis, particularly determining gene expression profiles in urine microvesicles.

### BACKGROUND

Increasing knowledge of the genetic and epigenetic changes occurring in cancer cells provides an opportunity to detect, characterize, and monitor tumors by analyzing tumor-related nucleic acid sequences and profiles. These changes can be observed by detecting any of a variety of cancer-related biomarkers. Various molecular diagnostic assays are used to detect these biomarkers and produce valuable information for patients, doctors, clinicians and researchers. So far, these assays primarily have been performed on cancer cells derived from surgically removed tumor tissue or from tissue obtained by biopsy.

However, the ability to perform these tests using a bodily fluid sample is oftentimes more desirable than using a patient tissue sample. A less invasive approach using a bodily fluid sample has wide ranging implications in terms of patient welfare, the ability to conduct longitudinal disease monitoring, and the ability to obtain expression profiles even when tissue cells are not easily accessible, e.g., in the prostate gland. For these samples, the collection methods previously disclosed often required a digital rectal exam (DRE) or prostate massage to enable enough prostate-derived cellular fluid to enter the urine. Samples collected without DRE or prostate massage showed a lower detection rate of these biomarkers.

Accordingly, there exists a need for new, noninvasive methods of detecting biomarkers, for example, biomarkers in urinary microvesicles, to aid in diagnosis, prognosis, monitoring, or therapy selection for a disease or other medical condition of the prostate gland. In particular, there exists a need for noninvasive methods that do not require DRE or prostate massage prior to urine sample collection and do not require a sample preparation step involving isolation of a cellular pellet from urine samples.

### SUMMARY OF THE INVENTION

The present invention provides methods of detecting one or more biomarkers in urine microvesicles to aid in diagnosis or monitoring of prostate cancer in a subject.

Cancer-related biomarkers include, e.g., specific mutations in gene sequences (Cortez and Calin, 2009; Diehl et al., 2008; Network, 2008; Parsons et al., 2008), up- and down-regulation of mRNA and miRNA expression (Cortez and Calin, 2009; Itadani et al., 2008; Novakova et al., 2009), mRNA splicing variations, changes in DNA methylation patterns (Cadieux et al., 2006; Kristensen and Hansen, 2009), amplification and deletion of genomic regions (Cowell and Lo, 2009), and aberrant expression of repeated DNA sequences (Ting et al., 2011). Various molecular diagnostic assays such as mutational analysis, methylation status of genomic DNA, and gene expression analysis may detect these biomarkers and produce valuable information for patients, doctors, clinicians and researchers. So far, these assays primarily have been performed on cancer cells derived from surgically removed tumor tissue or from tissue obtained by biopsy. For example, PCA3, TMPRSS2:ERG, and ERG, have previously been shown through biopsy analysis to be differentially expressed in prostate cancer compared to normal prostate tissues (Bussemakers et al., 1999; Petrovics et al., 2005; Tomlins et al., 2005).

However, the ability to perform these tests using a bodily fluid sample is oftentimes more desirable than using a patient tissue sample. A less invasive approach using a bodily fluid sample has wide ranging implications in terms of patient welfare, the ability to conduct longitudinal disease monitoring, and the ability to obtain expression profiles even when tissue cells are not easily accessible, e.g., in the prostate gland.

The detection of prostate cancer markers such as PSA (also called KLK3), PCA3, TMPRSS2:ERG, and ERG using urine samples has previously been investigated (Hessels et al., 2007; Laxman et al., 2008; Laxman et al., 2006; Nguyen et al., 2011; Rice et al., 2010; Rostad et al., 2009; Salami et al., 2011; Tomlins et al., 2005). However, the sample collection methods previously disclosed required a digital rectal exam (DRE), or prostate massage, to enable enough prostate-derived cellular fluid to enter the urine. Samples collected without DRE or prostate massage showed a lower detection rate of these biomarkers. For example, the detection rate for TMPRSS2:ERG was about 69% with DRE but only about 24% without DRE (Rostad et al., 2009).

Indeed, current sample collection methods for urine analysis of prostate cancer biomarkers require the use of a DRE with a systematic application of mild digital pressure over the entire palpated surface of the prostate, digital pressure to the prostate with 3 sweeps of each lateral lobe, firm pressure to the prostate from the base to apex and from the lateral to the median line of each lobe, or firm pressure to the prostate from the base to apex and from the lateral to the median line (where the depression of the prostate surface was between 0.5 to 1 cm) of each lobe three times (Deras et al., 2008; Hessels et al., 2007; Laxman et al., 2008; Laxman et al., 2006; Nguyen et al., 2011; Rice et al., 2010; Salami et al., 2011).

In addition, sample preparation methods previously disclosed require the isolation of cellular pellets from the post-DRE urine sample by centrifugation (Hessels et al., 2007; Laxman et al., 2008; Laxman et al., 2006; Nguyen et al., 2011; Rostad et al., 2009; Salami et al., 2011).

Many prior studies suggest that a DRE is a critical step in enabling enough RNA material to be collected for non-invasive prostate gene analysis (Deras et al., 2008; Hessels et al., 2007; Laxman et al., 2008; Laxman et al., 2006; Nguyen et al., 2011; Rice et al., 2010; Rostad et al., 2009; Salami et al., 2011; Tomlins et al., 2011). In some of these studies, urine samples are required to be processed within 4 hours of collection (Deras et al., 2008; Tomlins et al., 2011).

Nilsson J. et al., "Prostate cancer-derived urine exosomes: a novel approach to biomarkers for prostate cancer", British Journal of Cancer, Nature Publishing Group, vol. 100, no. 10, 1 May 2009, pages 1603-1607, discloses microvesicles from urine of suspected prostate cancer patients and controls before and after digital rectal examination/prostatic massage. PCA3 and ERG expression and fusion products are detected. WO 2009/126122 A1 discloses the detection of prostate cancer in urine derived microvesicles using specific ERG isoforms, detecting the expression of combinations of the PSA/KLK3, PMEPA1, NKX3.1, ODC1, AMD1 and ERG genes. WO 2005/113816 A2 discloses urine tests for prostate cancer detecting, amongst others, isoforms of ERG, PCA3. It is silent as to whether prostatic massage is used and as to whether microvesicles were analyzed. WO 2010/062706 A2 discloses a similar method and is also silent as to whether prostatic massage is used.

In contrast to these previous sample collection and urinary biomarker detection methods, the methods provided herein do not require a DRE or prostate massage prior to urine sample collection, nor do these methods require a sample preparation step involving isolation of a cellular pellet from urine samples. These new, noninvasive methods use urinary microvesicles to detect biomarkers in aid of diagnosis, prognosis, monitoring, or therapy selection for a disease or other medical condition of the prostate gland. Microvesicles released by tumor cells can be used to determine the genetic status of the tumor (Skog et al., 2008). See also WO 2009100029, WO 2011009104, WO 2011031892, and WO 2011031877.

In a first aspect of the invention there is provided a method for diagnosis or monitoring of prostate cancer in a subject, comprising the steps of:
(a) obtaining a microvesicle fraction from a urine sample from a subject, where the sample is collected without the use of a digital rectal exam (DRE) or prostatic massage prior to urine collection;
(b) extracting one or more nucleic acids from the microvesicle fraction; and
(c) analyzing the extracted nucleic acids to detect the level of expression of two biomarkers associated with prostate cancer, wherein the first biomarker is one or more isoforms of ERG and the second biomarker is PCA3.

Preferred embodiments of the invention in any of its various aspects are as described below or as defined in the sub claims.

The invention provides a method for diagnosis, or monitoring of prostate cancer in a subject, comprising the steps of: (a) obtaining a microvesicle fraction from a urine sample from a subject; (b) extracting one or more nucleic acids from the microvesicle fraction; and (c) analyzing the extracted nucleic acids to detect the presence or absence of two biomarker associated with prostate cancer, wherein the first biomarker is one or more isoforms of ERG and the second biomarker is PCA3.

In some embodiments, the RNA expression profile is a combination of an RNA expression profile of one or more isoforms of the ERG gene and an RNA expression profile of PCA3.

### BRIEF DESCRIPTION OF THE DRAWINGS

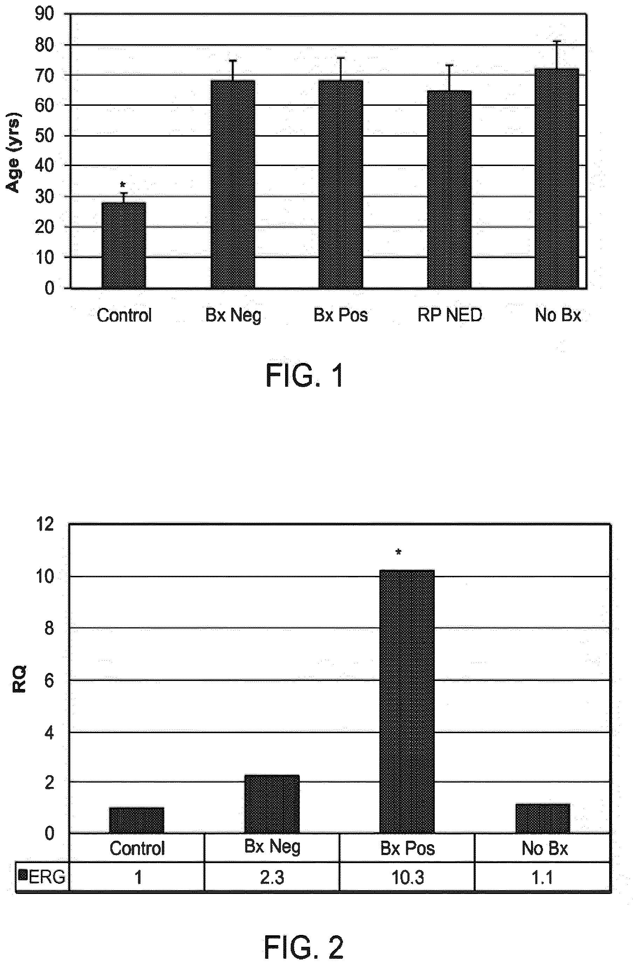

Figure 1 is a bar chart depicting the statistical analysis results of each subject's age in five groups of individuals in the study here disclosed. These data show that the subjects in the control group (Control, males under 35 years old) were statistically significantly younger than the subjects in each of the other four groups: biopsy positive (Bx Pos), biopsy negative (Bx Neg), radical prostatectomy no evidence of disease (RP NED), and patients without a biopsy (No Bx). The asterisk indicates *P*<0.001 for the Control group versus each of the other four groups. Control n= 40, Bx Neg n= 39, Bx Pos n=48, RP NED n=35, No Bx n=44. "N" refers to the number of subjects in the group.
Figure 2 is a chart showing the RQ analysis results of ERG expression in four of the five groups referenced in Figure 1, namely Control, Bx Neg, Bx Pos, and No Bx. Here, the Control group was used as the calibrator group in the RQ analysis, and the GAPDH gene was the reference gene in the RQ analysis, i.e., the ERG gene expression level was standardized to GAPDH gene expression. The Y axis represents the RQ value as derived by the DATA Assist Program. The asterisk indicates *P*=0.0074 for the Bx Pos versus the Control group. In Figures 2-5, the RQ analysis of the expression data was performed using the Data Assist program (obtained Applied BioSystems).

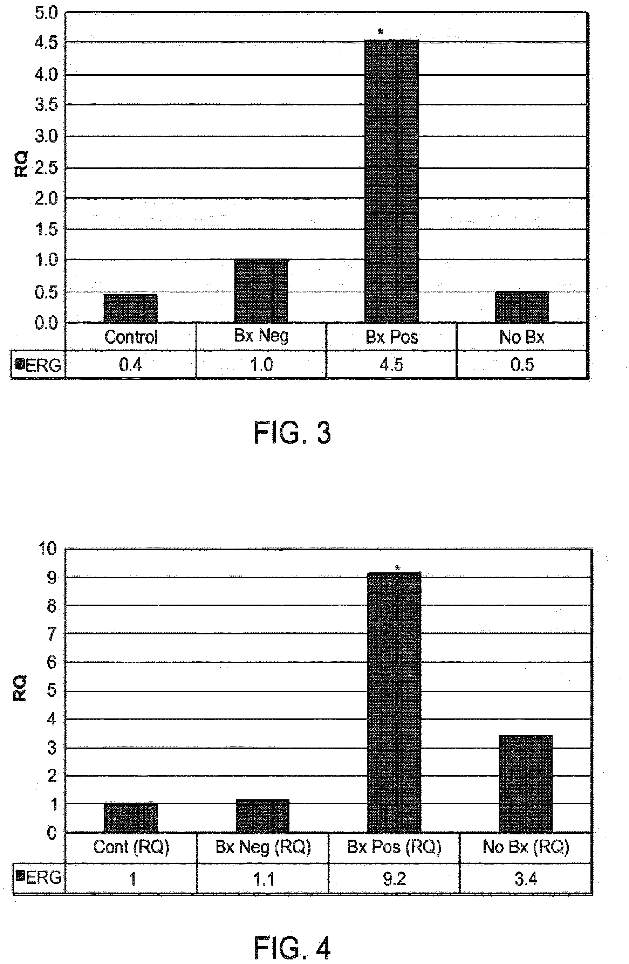

Figure 3 is a chart showing the RQ analysis results of ERG expression as in Figure 2, except that the age-matched `Bx Neg' (biopsy negative) group was used as the calibrator group. As in Figure 2, the GAPDH gene was the reference gene in the RQ analysis, i.e., the ERG gene expression level was standardized to GAPDH gene expression. The asterisk indicates *P*= 0.0236 for the Bx Pos versus the Bx Neg group.
Figure 4 is a chart showing the RQ analysis results of ERG expression as in Figure 2, except that the PSA (KLK3) gene was the reference gene in the relative quantitation analysis, i.e., the ERG gene expression level was standardized to PSA gene expression. The asterisk indicates *P*=0.0049 for the Bx Pos versus the Control group.

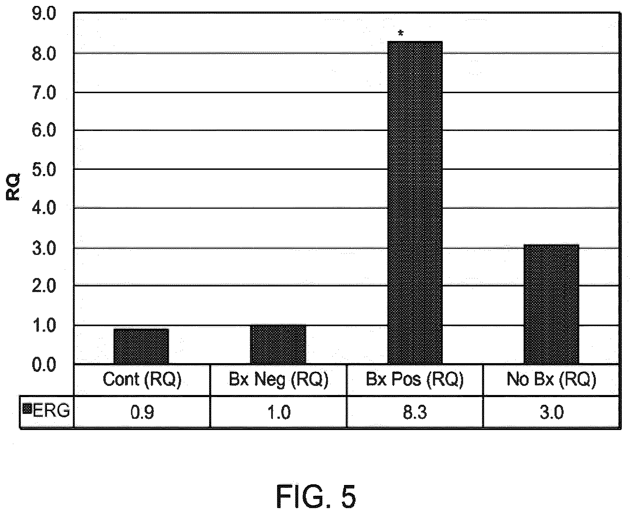

Figure 5 is a chart showing the RQ analysis results of ERG expression as in Figure 3, except that the PSA gene was the reference gene in the relative quantitation analysis, i.e., the ERG gene expression level was standardized to PSA gene expression. The asterisk indicates *P*= 0.0025 for the Bx Pos versus the Bx Neg group.

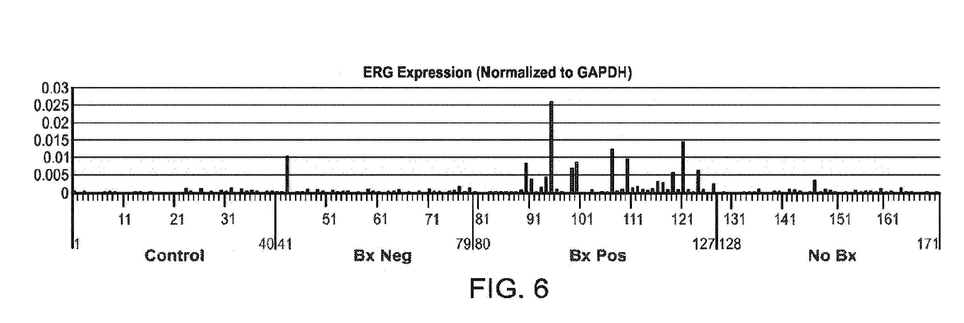

Figure 6 is a chart showing the ERG expression level in each individual of the four groups (Control, Bx Neg, Bx Pos, and No Bx) mentioned in Figure 1. GAPDH was the reference gene in the RQ analysis, i.e., the ERG gene expression level was standardized to GAPDH gene expression. The ERG expression level is represented as 2 to (-Delta CT), as defined in RQ analysis (see detailed description of the analysis below) using the Data Assist program (obtained from Applied BioSystems).

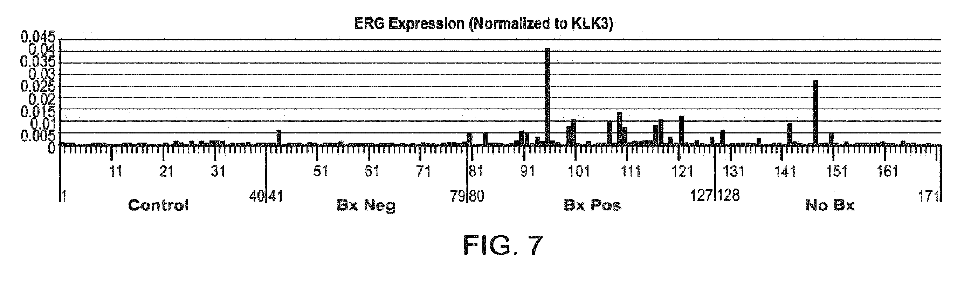

Figure 7 is a chart showing ERG expression levels as in Figure 6, except that PSA gene was the reference gene in the RQ analysis, i.e., the ERG gene expression level was standardized to PSA gene expression. The ERG expression level is represented as 2 to (-Delta CT) as defined in RQ analysis using the Data Assist program (obtained from Applied BioSystems).

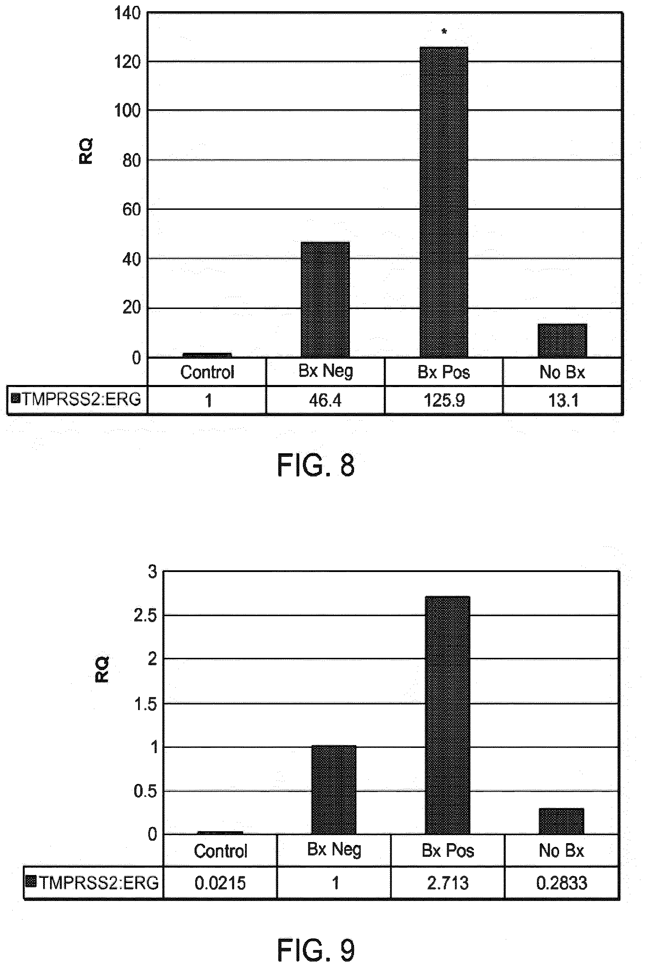

Figure 8 is a chart showing the RQ analysis results of TMPRSS2:ERG fusion gene expression in the four of the five groups, namely Control, Bx Neg, Bx Pos, and No Bx, as mentioned in Figure 1. Here, the Control group was used as the calibrator group in the RQ analysis, and the GAPDH gene was the reference gene in the relative quantitation analysis, i.e., the TMPRSS2:ERG fusion gene expression level was standardized to GAPDH gene expression. The asterisk indicates *P*=0.0008 for the Bx Pos versus the Control group. The relative quantitation analysis of the expression data was performed using the Data Assist program (ABI).
Figure 9 is a chart showing the RQ analysis results of TMPRSS2:ERG fusion gene expression as in Figure 8, except that the PSA gene was the reference gene in the relative quantitation analysis, i.e., the TMPRSS2:ERG fusion gene expression level was standardized to PSA gene expression. The asterisk indicates *P*=0.0007 for the Bx Pos versus the Control group.

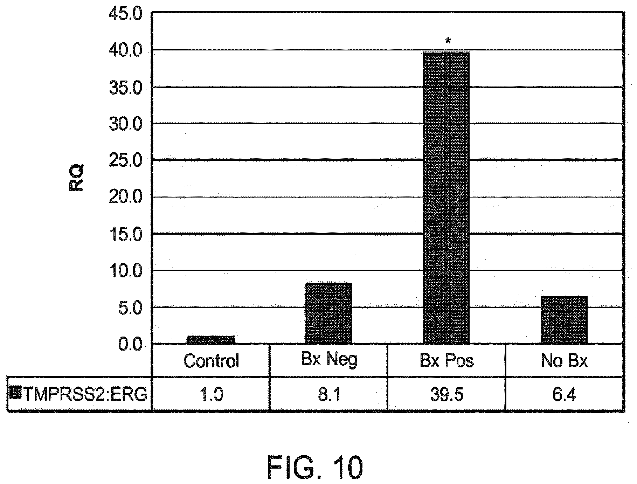

Figure 10 is a chart showing the RQ analysis results of TMPRSS2:ERG fusion gene expression as in Figure 8 except that the Bx Neg group was the calibrator group. The asterisk indicates *P*=*0.1171* for the Bx Pos versus the Bx Neg group.

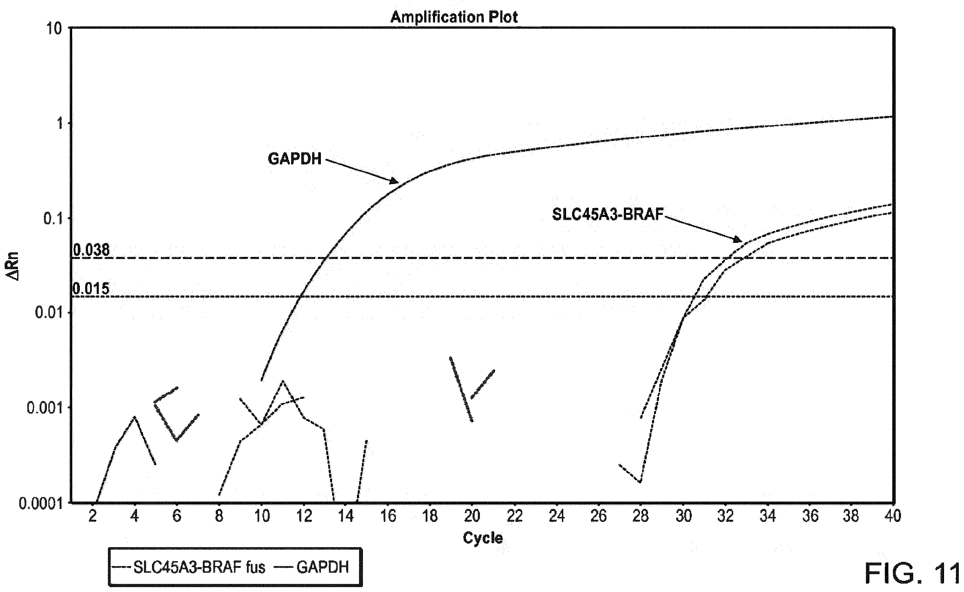

Figure 11 depicts a plot showing SLC45A3:BRAF amplification curves from RT-PCR. The X axis represents the number of PCR amplification cycles. The Y axis represents the ΔRn, which is the magnitude of the signal generated by the given set of PCR conditions. The number 0.038 on the Y axis is the threshold line for GAPDH. The number 0.015 on the Y axis is the threshold for SLC45A3:BRAF. A threshold line is the line whose intersection with the amplification plot defines the threshold cycle (Ct) in real-time PCR assays. The level is set above the baseline, but sufficiently low to be within the exponential growth region of the PCR amplification curve.

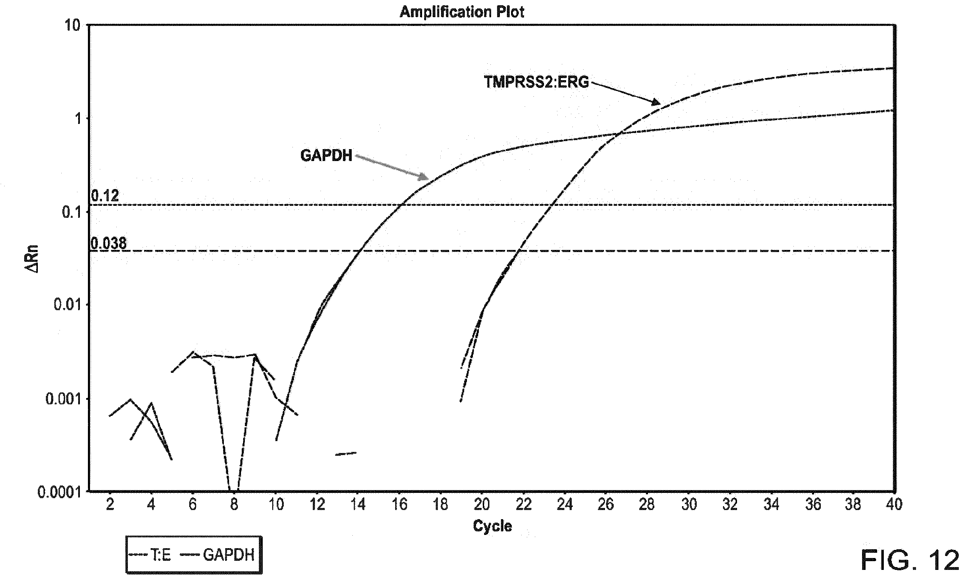

Figure 12 depicts a plot showing TMPRSS2-ERG amplification curves from RT-PCR with the same RNA as in Figure 12. The X axis represents the number of PCR amplification cycles. The Y axis represents the ΔRn, which is the magnitude of the signal generated by the given set of PCR conditions. The number 0.038 on the Y axis is the threshold line for GAPDH. The number 0.12 on the Y axis is the threshold for TMPRSS2:ERG.

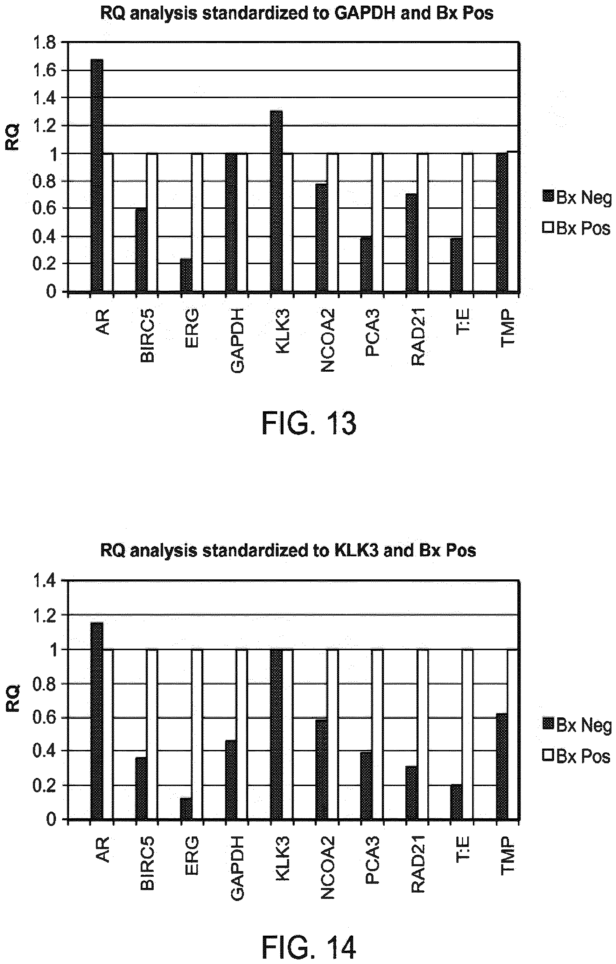

Figure 13 is a chart depicting the RQ analysis results of the ten genes in the Bx Pos and Bx Neg groups using GAPDH as the reference gene. The ten genes were androgen receptor (AR), BIRC5 (survivin), ERG, GAPDH, KLK3 (PSA), NCOA2, PCA3, RAD21, TMPRSS2:ERG, and TMPRSS2.
Figure 14 is a chart depicting the RQ analysis results of the ten genes in the Bx Pos and Bx Neg groups as in Figure 13 except that PSA was used as the reference gene.

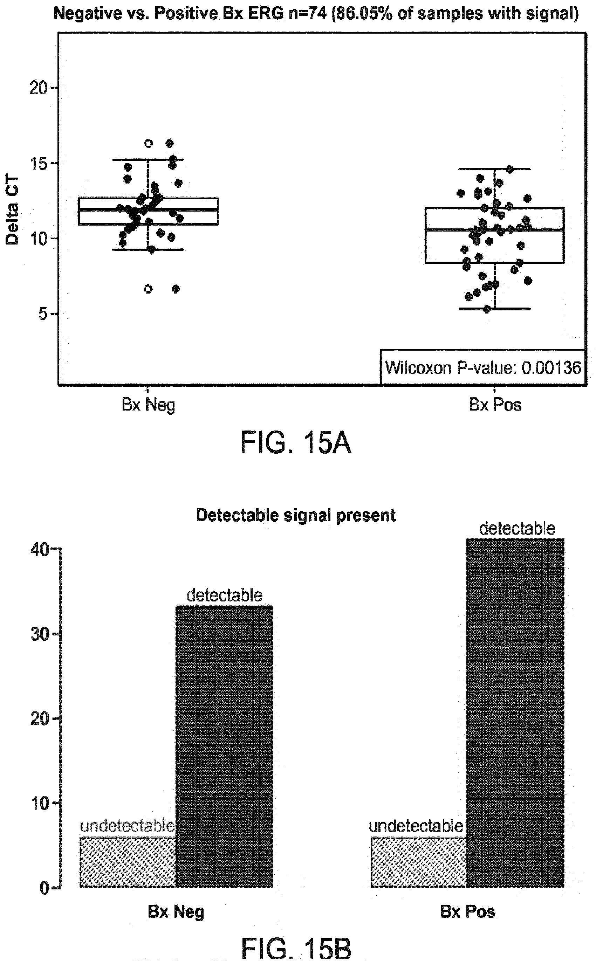

Figure 15 depicts an ERG gene expression analysis using the Delta Ct method. A. A strip plot of ERG expression levels in the Bx Neg and Bx Pos groups. The Y axis is the Delta Ct value between ERG and the reference gene GAPDH. B. A chart showing the number of patients in which the ERG expression were detectable or undetectable.

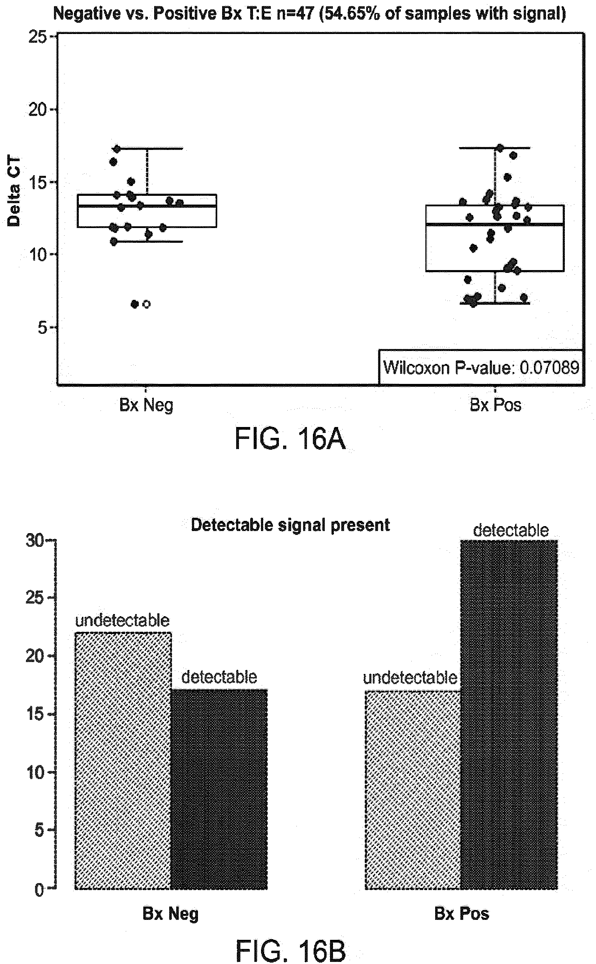

Figure 16 depicts a TMPRSS2:ERG fusion gene expression analysis using the Delta Ct method. A. A strip plot of TMPRSS2:ERG expression levels in the BxNeg and BxPos groups. The Y axis is the Delta Ct value between TMPRSS2:ERG and the reference gene GAPDH. B. A chart showing the number of patients in which the TMPRSS2:ERG expression were detectable or undetectable.

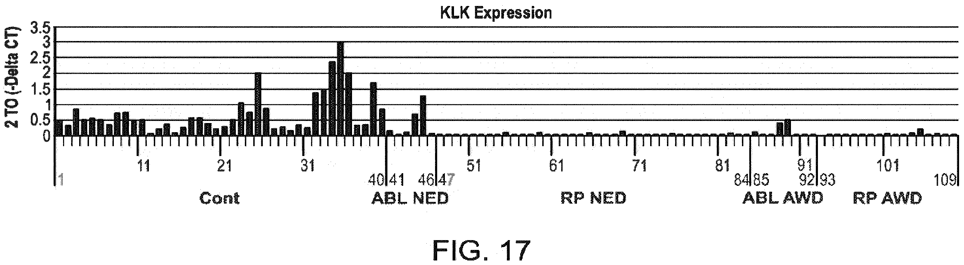

Figure 17 is a chart depicting the expression levels of PSA in five different groups of patients. The five groups are: the Control group with healthy individuals (Cont), the group of patients who had undergone ablation therapy but exhibited no evidence of disease (ABL NED), the group of patients who had undergone radical prostatectomy but exhibited no evidence of disease (RP-NED), the group of patients who had undergone ablation therapy and were alive with disease (ABL AWD), and the group of patients who had undergone radical prostatectomy and were alive with disease (RP-AWD). The Y axis is the expression levels represented by the 2^{-ΔCt}. The X axis is the individual patient designated with an indexed number in the five groups.

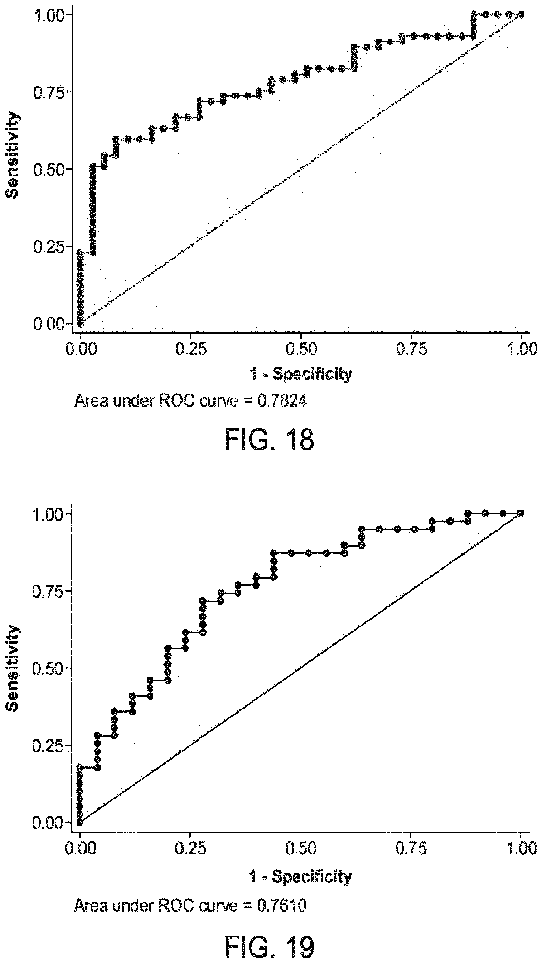

Figure 18 is a chart showing ROC curves based on ERG expression analysis with urine samples from Biopsy positive and negative prostate cancer patients. The X axis refers to specificity. The Y axis refers to sensitivity.
Figure 19 is a chart showing ROC curves based on AMACR expression analysis similar to the one in Figure 18.

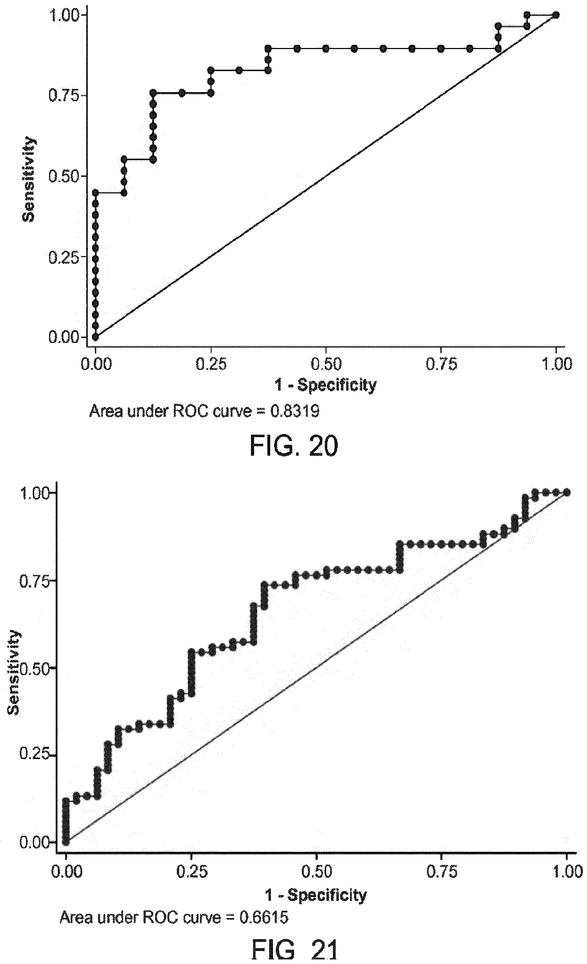

Figure 20 is a chart showing ROC curves based on both ERG and AMACR expression analysis similar to the one in Figure 18.
Figure 21 is a chart showing ROC curves based on PCA3 expression analysis similar to the one in Figure 18.

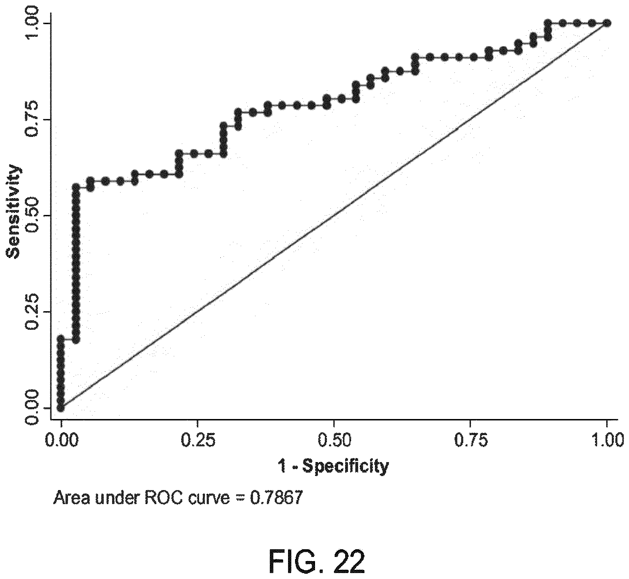

Figure 22 is a chart showing ROC curves based on both ERG and PCA3 expression analysis similar to the one in Figure 18.

### DETAILED DESCRIPTION

The present invention is based on the surprising finding that urine microvesicles contain biomarkers for a disease or other medical condition of the prostate gland in a subject. Thus, a patient urine sample can be assayed for detection of biomarkers for a disease or other medical condition of the prostate gland in a subject.

In the methods provided herein, urine samples from subjects are collected without using a digital rectal exam (DRE) or prostatic massage prior to urine collection. In the methods provided herein, urine samples are first pre-processed by using a method comprising at least one filtration step. For example, a course filter (0.8 micron) is utilized to remove cells and cell debris. This filtration may be followed by an ultrafiltration step to remove solvent and small molecule analytes while retaining the microvesicles. The filters used in the initial filtration can be any size that is sufficient to remove cells and cell debris, for example, any size greater than 0.22 microns. To isolate the urine microvesicles, the pre-processed samples are then subjected to a filtration concentration step, wherein a filter that has a molecular weight cutoff is utilized to retain and concentrate the microvesicles that are greater than 10 nm in diameter. For example, the sample is then concentrated to a volume of less than 1 ml, preferably 100-200 ul. For example, the molecular weight cutoff is at least 100 kDa. Preferably, the molecular weight cutoff is 100 kDa.

After isolation and concentration of the urine microvesicles, the samples are pre-treated with an RNase inhibitor, prior to nucleic acid extraction, to prevent digestion of extracted RNA and enhance the quality of the extraction. RNA is extracted from the microvesicles by a method comprising lysis of the microvesicles, processing the lysate through an RNA-binding column, and elution of the RNA from the RNA-binding column, under appropriate conditions designed to achieve high quality RNA preparations.

These high quality RNA preparations provide urine-based molecular diagnostics for prostate cancer and other disorders of the prostate.

The methods provided herein are useful in subjects suspected of having prostate cancer, for example, due to an elevated PSA, suspicious DRE or any other art-recognized technique for diagnosis of prostate cancer.

The methods provided herein demonstrate the association of biomarkers in urine microvesicles with the finding of prostate cancer as determined by a prostate biopsy. Prostate biopsy is the current standard for prostate cancer diagnosis, but the risks associated with prostate biopsy are significant, especially when considering that one million biopsies are performed in the United States, annually. Pain, bleeding, urinary retention and urinary tract infections are not uncommon, and serious life threatening infections may also occur.

The methods described herein provide methods of the non-invasive analysis of the RNA expression levels of prostate cancer-associated transcripts in urinary microvesicles. In particular, the methods are used to detect the mRNA expression of at least one or more isoforms of AMACR (α-methylacyl-coenzyme A racemase, an enzyme that interconverts pristanoyl-CoA and C27-bile acylCoAs between their (R)- and (S)-stereoisomers) and one or more isoforms of ERG (E-twenty six (ETS) related gene) in urinary microvesicles. The one or more isoforms of ERG include ERG1, ERG2, ERG3, ERG4, ERG5, ERG6, ERG7, ERG8, ERG9, ERG Prostate Cancer-specific Isoform 1 (EPC1) and ERG Prostate Cancer-specific Isoform 2 (EPC2). As demonstrated herein, detecting expression levels of at least ERG and AMACR in urinary microvesicles provides excellent sensitivity and specificity as biomarkers of prostate cancer and other prostate-related disorders in subjects who had previously undergone a prostate biopsy (referred to herein as the biopsy cohort).

In the methods provided herein, the level of mRNA expression of both AMACR and at least one isoform of ERG is detected. The level of mRNA expression is detecting using any of a variety of art-recognized techniques. For example, the Ct (cycle threshold) values for each biomarker in urine microvesicles are determined by RT-qPCR analysis of a urine exosomal RNA concentrate. In a real time PCR assay a positive reaction is detected by accumulation of a fluorescent signal. The Ct value is defined as the number of cycles required for the fluorescent signal to cross the threshold (i.e., exceeds background level). Ct levels are inversely proportional to the amount of target nucleic acid in the sample (i.e., the lower the Ct level the greater the amount of target nucleic acid in the sample).

The expression levels of additional genes can also be measured in the methods provided herein. For example, in the methods provided herein, a prostate-specific reference gene is also detected to demonstrate the relative expression levels of ERG and AMACR as compared to a prostate specific RNA gene expression level. For example, the mRNA expression level for KLK3, the gene encoding for prostate specific antigen (PSA) can also be measured. The mRNA expression level for any prostate-specific gene or GAPDH is measured. In the methods provided herein, the relative expression analysis is accomplished by subtracting the Ct value for the prostate-specific marker gene (e.g., KLK3) from ERG or AMACR, with the result referred to as ΔCt ERG and ΔCt AMACR, respectively.

Alternatively or in addition, the mRNA expression level of a gene typically found in urine microvesicles can also be measured to demonstrate the sufficiency of the urine sample for exosomally-derived RNA. For example, the mRNA expression level of GAPDH, a housekeeping gene encoding for glyceraldehyde 3-phosphate dehydrogenase can also be measured. The expression level of GAPDH can be used to determine sufficiency of the urine sample for exosomally-derived RNA, as all cells of the genitourinary system (kidney, bladder and prostate) shed microvesicles into the urine and contribute to the GAPDH level.

In the methods provided herein, those genes whose expression levels are used to calculate relative expression levels are referred to collectively as "reference genes." Suitable reference genes for determination of the sufficiency of the urine sample for exosomally-derived RNA are genes that are typically found in urine microvesicles, such as house-keeping genes or prostate-specific genes. The expression level of these reference genes are used to normalize for the amount of signal detected to control for variability in the quantity of microvesicles isolated between samples. For example, the reference gene is GAPDH. A reference gene for determination of the relative expression level is a prostate-specific gene. For example, the reference gene is KLK3 (PSA), which is a prostate-specific gene.

The relative expression levels of AMACR, ERG and the prostate-specific marker gene can also be analyzed and compared using any of a variety of art-recognized techniques. For example, Receiver Operating Characteristics (ROC) analysis can be conducted for any combination of AMACR, ERG and prostate-specific marker gene expression levels to yield an Area Under the Curve (AUC) for each biomarker measured. The ROC analyses of ERG and AMACR can be run individually, i.e., as individual biomarkers, or combined for linear regression analysis.

As shown in the examples provided herein, ERG is a sensitive biomarker that specifically differentiates between biopsy negative and biopsy positive subjects, with a 95% confidence interval in the range of 0.69-0.87 (Figure 18). AMACR is a sensitive biomarker that specifically differentiates between biopsy negative and biopsy positive subjects, with a 95% confidence interval in the range of 0.64-0.88 (Figure 19). AMACR and ERG together are sensitive combined biomarkers that specifically differentiate between biopsy negative and biopsy positive subjects, with a 95% confidence interval in the range of 0.71-0.95 (Figure 20). These values demonstrate the strength of AMACR, ERG, and the combination of AMACR and ERG as diagnostic biomarkers for prostate cancer.

One aspect of the invention is a method for analyzing nucleic acid biomarkers that originate from prostate cells using urine samples. The method may be used for purposes of aiding in diagnosis or monitoring of prostate cancer in a subject. In this method, one would obtain a microvesicle fraction from a urine sample from a subject, extract nucleic acids from the fraction, and analyze the extracted nucleic acids to detect the presence or absence of one or more biomarkers originating associated with a disease or other medical condition of the prostate gland.

In one embodiment, the step of obtaining a microvesicle fraction from a urine sample from a subject comprises the use of affinity selection to enrich for microvesicles having surface markers associated with prostate cells or tissues. In another embodiment, the step of obtaining a microvesicle fraction from a urine sample from a subject comprises the use of affinity exclusion to remove microvesicles having surface markers associated with cells or tissues that are not part of the prostate gland. In a further embodiment, the step of obtaining a microvesicle fraction from a urine sample from a subject comprises a combination of the techniques described above.

In the foregoing methods, the steps may be repeated over time when the purpose of the analysis is to monitor the progression of a disease or other medical condition, treatment efficacy, or the subject's overall health status. The frequency, as well as the total number, of repeats is discretionary. A person skilled in the art, e.g., a healthcare professional, may determine the frequency in a case-by-case basis. In other cases, a standard of care will determine the frequency of repeated monitoring.

The term "microvesicles" refers to cell-derived vesicles that are heterogeneous in size with diameters ranging from about 10 nm to about 1 µm . For example, "exosomes" have diameters of approximately 30 to 200 nm, with shedding microvesicles and apoptotic bodies often described as larger (Orozco and Lewis, 2010). Exosomes, shedding microvesicles, microparticles, nanovesicles, apoptotic bodies, nanoparticles and membrane vesicles may co-isolate using various techniques and are, therefore, collectively referred to throughout this specification as "microvesicles" unless otherwise expressly denoted.

In the foregoing methods, a urine sample from a subject may be obtained in many different ways. In some instances, a urine sample may be collected and subjected to the procedure in the method almost immediately. In other instances, a urine sample is collected and stored in an appropriate condition for future analysis. The storage condition may be in a 4°C environment or similar environment that does not significantly affect the quality of future microvesicle isolation, microvesicle fraction procurement, or nucleic acid extraction and biomarker analysis.

The term "subject" is intended to include all animals shown to or expected to have nucleic acid-containing microvesicles and/or circulating nucleic acids in urine. In particular embodiments, the subject is a mammal; for example, a human or nonhuman primate, a dog, a cat, a horse, a cow or another farm animal, or a rodent (e.g. a mouse, rat, guinea pig. etc.).

The quantity of the urine sample may vary depending on how much nucleic acid is needed for each analysis, how many times the analysis needs to be carried out, or how many different biomarkers need to be analyzed. The amount may be 1ml, 5ml, 10ml, 20ml, 50ml, 100ml, 200ml, 500ml, or any amount that is deemed necessary to obtain a desired analytical result. Generally, a sample of 20ml is used for microvesicle fraction procurement and nucleic acid extraction.

The timing for collecting urine samples may also vary depending on different applications. A sample may be collected at any anytime as a spot urine sample. Spot urine may be sufficient for biomarker analyses when the amount of biomarker in microvesicles to be analyzed does not fluctuate too much during the day. In other cases, a 24-hour urine sample is collected when there is fluctuation of the amount of the biomarker in microvesicles to be analyzed and a 24-hour collection may mitigate the fluctuation effect. In still further cases, a series of urine samples are collected to study the fluctuation of the amount of biomarkers in microvesicles. The series of collections may be carried out in a certain time interval, e.g., every 6 hours, or in a scenario interval, e.g., before and after a therapeutic intervention.

### Procurement of a microvesicle fraction from a urine sample

Methods for procuring a microvesicle fraction from a urine sample are described in this application as well as in scientific publications and patent applications (Chen et al., 2010; Miranda et al., 2010; Skog et al., 2008). See also WO 2009/100029, WO 2011009104, WO 2011031892, and WO 2011031877. These publications disclose microvesicle isolation or fraction procurement methods and techniques.

For example, methods of microvesicle procurement by differential centrifugation are described in a paper by Raposo et al. (Raposo et al., 1996), a paper by Skog et al.(Skog et al., 2008) and a paper by Nilsson et. al.(Nilsson et al., 2009). Methods of anion exchange and/or gel permeation chromatography are described in US Patent Nos. 6,899,863 and 6,812,023. Methods of sucrose density gradients or organelle electrophoresis are described in U.S. Patent No. 7,198,923. A method of magnetic activated cell sorting (MACS) is described in a paper by Taylor and Gercel-Taylor (Taylor and Gercel-Taylor, 2008). A method of nanomembrane ultrafiltration concentration is described in a paper by Cheruvanky et al. (Cheruvanky et al., 2007). Further, microvesicles can be identified and isolated from a subject's bodily fluid by a microchip technology that uses a microfluidic platform to separate tumor-derived microvesicles (Chen et al., 2010).

In one embodiment of the methods described herein, the microvesicles isolated from urine are enriched for those originating from prostate or tumor cells. Because the microvesicles often carry surface molecules such as antigens from their donor cells, surface molecules may be used to identify, isolate and/or enrich for microvesicles from a specific donor cell type (Al-Nedawi et al., 2008; Taylor and Gercel-Taylor, 2008). In this way, microvesicles originating from distinct cell populations can be analyzed for their nucleic acid content. For example, tumor (malignant and non-malignant) microvesicles carry tumor-associated surface antigens and may be detected, isolated and/or enriched via these specific tumor-associated surface antigens. In one example, the surface antigen is epithelial-cell-adhesion-molecule (EpCAM), which is specific to microvesicles from carcinomas of lung, colorectal, breast, prostate, head and neck, and hepatic origin, but not of hematological cell origin (Balzar et al., 1999; Went et al., 2004).

Additionally, tumor specific microvesicles may be characterized by the lack of surface markers, such as CD80 and CD86. In these cases, microvesicles with the markers, such as CD80 and CD86, may be excluded for further analysis of tumor specific markers. The exclusion may be achieved by various methods, for example, affinity exclusion.

The procurement of microvesicle fractions from prostate can be accomplished, for example, by using antibodies, aptamers, aptamer analogs or molecularly imprinted polymers specific for a desired surface antigen. In one embodiment, the surface antigen is specific for a cancer type. In another embodiment, the surface antigen is specific for a cell type which is not necessarily cancerous.

One example of a method of microvesicle separation based on cell surface antigen is provided in U.S. Patent No. 7,198,923. As described in, e.g., U.S. Patent Nos. 5,840,867 and 5,582,981, WO/2003/050290 and a publication by Johnson et al. (Johnson et al., 2008), aptamers and their analogs specifically bind surface molecules and can be used as a separation tool for retrieving cell type-specific microvesicles. Molecularly imprinted polymers also specifically recognize surface molecules as described in, e.g., US Patent Nos. 6,525,154, 7,332,553 and 7,384,589 and a publication by Bossi et al. (Bossi et al., 2007) and are a tool for retrieving and isolating cell type-specific microvesicles.

In the methods described herein, a urine sample may be pre-processed by one or more filtration or centrifugation steps to remove cell debris and other non-microvesicle matter. For example, the urine sample may be filtered through a 0.8 um filter. Optionally, the filtrate acquired from the 0.8 um filter may be further filtered through a 0.22 um filter. To isolate the urine microvesicles, the pre-processed samples are then concentrated using a filtration concentration step. This step comprises utilizing a filter that has a molecular cutoff to retain and concentrate the microvesicles that are greater than 10 nm in diameter. For example, the sample is then concentrated to a volume of less than 1 ml, preferably 100-200 ul. For example, the molecular weight cutoff is at least 100 kDa. Preferably, the molecular weight cutoff is 100 kDa.

### Nucleic acid extraction from microvesicles

Methods for nucleic acid extraction are generally based on procedures well-known in the art. Persons of skill will select a particular extraction procedure as appropriate for the particular biological sample. Examples of extraction procedures are provided in patent publications WO/2009/100029, US 20100196426, US 20110003704, US 20110053157, WO 2011009104, and WO 2011031892. These publications disclose microvesicle nucleic acid extraction methods and techniques.

In the methods described herein, an RNase inhibitor is added to the sample after microvesicle isolation and purification, but prior to microvesicle lysis and nucleic acid extraction for the purpose of preventing undesirable degradation of the nucleic acids after extraction. The microvesicles are lysed in the present of RNase inhibitor. The lysate is then added to an RNA-binding column, under such conditions known in the art so that the microvesicle RNA binds to the column. Optionally, the column is washed to increase the quality and yield of the RNA. Then the RNA is eluted under conditions known in the art such that high quality RNA is collected.

### Detection of nucleic acid biomarkers

Biomarker detection can be carried out on the extracted nucleic acids in many different ways and constitute many aspects. In some embodiments, the detection of nucleic acid biomarkers from one or more urine samples is to obtain a profile of all or portions of the extracted nucleic acids.

A profile, as the term is used herein, refers to a representation of particular features of a collection of nucleic acids, which can be determined through the quantitative or qualitative analysis of one or more nucleic acids contained in microvesicles isolated from a urine sample from a subject. A reference profile is here defined as a profile obtained from an independent subject or a group of subject, or from the same subject at a different time point.

The nucleic acids in microvesicles can be one or more types of nucleic acids, examples of which are provided herein.

The nucleic acids can be RNA. RNA can be coding RNA, e.g., messenger RNA which may encode proteins. RNA can also be non-coding RNA (ncRNA), e.g., ribosomal RNA, transfer RNA, microRNA, and other non-coding transcripts that may originate from genomic DNA. These non-coding RNA transcripts may include transcripts that are transcribed from satellite repeats; and transposons which may be DNA transposons or retrotransposons.

The nucleic acids can be DNA. DNA can be single-stranded DNA, that is reverse transcribed from RNA, e.g., cDNA. Reverse transcription is usually mediated by reverse transcriptase encoded by a reverse transcriptase gene in a cell. The DNA can also be single stranded DNA that is generated during DNA replication. Genomic DNA replicates in the nucleus while the cell is dividing. Some of the replicated DNA may come off its template, be exported out of the nucleus, and packaged in microvesicles. The DNA can further be fragments of double-stranded DNA.

In addition, the DNA can be non-coding DNA (ncDNA). The human genome only contains about 20,000 protein coding genes, representing less than 2% of the genome. The ratio of non-coding to protein-coding DNA sequences increases as a function of developmental complexity (Mattick, 2004). Prokaryotes have less than 25% ncDNA, simple eukaryotes have between 25-50%, more complex multicellular organisms like plants and animals have more than 50% ncDNA, with humans having about 98.5% ncDNA (Mattick, 2004)

Some of the ncDNA from the genome are transcribed into ncRNAs. NcRNAs have been implicated in many important processes in the cell, e.g., enzymes (ribozymes), binding specifically to proteins (aptamers), and regulating gene activity at both the transcriptional and post-transcriptional levels.

A profile of nucleic acids can be obtained through analyzing nucleic acids obtained from isolated microvesicles according to standard protocols in the art. For example, the analysis of the DNA may be performed by one or more various methods known in the art, including microarray analysis for determining the nucleic acid species in the extract, quantitative PCR for measuring the expression levels of genes, DNA sequencing for detecting mutations in genes, and bisulfite methylation assays for detecting methylation pattern of genes.

To obtain profiles, in some instances, data analysis may be performed. Such data analysis can be performed, for example, by Clustering Analysis, Principle Component Analysis, Linear Discriminant Analysis, Receiver Operating Characteristic Curve Analysis, Binary Analysis, Cox Proportional Hazards Analysis, Support Vector Machines and Recursive Feature Elimination (SVM-RFE), Classification to Nearest Centroid, Evidence-based Analysis, or a combination of any of the foregoing analytical techniques.

For another example, the analysis of RNA may carried out using the Digital Gene Expression (DGE) analysis method (Lipson et al., 2009). For yet another example of RNA analysis, the RNA may be digested and converted into single stranded cDNA which may then be subject to sequencing analysis on a DNA sequencing machine, e.g., the HeliScope^{™} Single Molecule Sequencer from Helicos BioSciences as described in a publication by Ting et al. (Ting et al., 2011).

In other instances, the RNA may be reverse-transcribed into complementary DNA (cDNA) before further amplification. Such reverse transcription may be performed alone or in combination with an amplification step. One example of a method combining reverse transcription and amplification steps is reverse transcription polymerase chain reaction (RT-PCR), which may be further modified to be quantitative, e.g., quantitative RT-PCR as described in US Patent No. 5,639,606. Another example of the method comprises two separate steps: a first step of reverse transcription to convert RNA into cDNA and a second step of quantifying the amount of cDNA using quantitative PCR.

Nucleic acid amplification methods include, without limitation, polymerase chain reaction (PCR) (US Patent No. 5,219,727) and its variants such as in situ polymerase chain reaction (US Patent No. 5,538,871), quantitative polymerase chain reaction (US Patent No. 5,219,727), nested polymerase chain reaction (US Patent No. 5,556,773), self-sustained sequence replication and its variants (Guatelli et al., 1990), transcriptional amplification system and its variants (Kwoh et al., 1989), Qb Replicase and its variants (Miele et al., 1983), cold-PCR (Li et al., 2008), BEAMing (Li et al., 2006) or any other nucleic acid amplification methods, followed by the detection of the amplified molecules using techniques well known to those of skill in the art. Especially useful are those detection schemes designed for the detection of nucleic acid molecules if such molecules are present in very low numbers. In another embodiment, the step of nucleic acid amplification is not performed. Instead, the extracted nucleic acids are analyzed directly, e.g., through next-generation sequencing.

The analysis of nucleic acids present in the isolated microvesicles can be quantitative and/or qualitative. For quantitative analysis, the amounts (expression levels), either relative or absolute, of specific nucleic acids of interest within the isolated microvesicles are measured with methods known in the art (described above). For qualitative analysis, the species of nucleic acids of interest within the isolated microvesicles, whether wild type or variants, are identified with methods known in the art.

In other embodiments, the detection of nucleic acid biomarkers involves detection of the presence or absence of one or a collection of genetic aberrations. The term "genetic aberration" is used herein to refer to the nucleic acid amounts as well as nucleic acid variants within the nucleic acid-containing microvesicles. Specifically, genetic aberrations include, without limitation, over-expression of a gene (e.g., an oncogene) or a panel of genes, under-expression of a gene (e.g., a tumor suppressor gene such as p53 or RB) or a panel of genes, alternative production of splice variants of a gene or a panel of genes, gene copy number variants (CNV) (e.g., DNA double minutes) (Hahn, 1993), nucleic acid modifications (e.g., methylation, acetylation and phosphorylations), single nucleotide polymorphisms (SNPs) (e.g., polymorphisms in Alu elements), chromosomal rearrangements (e.g., inversions, deletions and duplications), and mutations (insertions, deletions, duplications, missense, nonsense, synonymous or any other nucleotide changes) of a gene or a panel of genes, which mutations, in many cases, ultimately affect the activity and function of the gene products, lead to alternative transcriptional splice variants and/or changes of gene expression level, or combinations of any of the foregoing.

Genetic aberrations can be found in many types of nucleic acids. The determination of such genetic aberrations can be performed by a variety of techniques known to the skilled practitioner. For example, expression levels of nucleic acids, alternative splicing variants, chromosome rearrangement and gene copy numbers can be determined by microarray analysis (see, e.g., US Patent Nos. 6,913,879, 7,364,848, 7,378,245, 6,893,837 and 6,004,755) and quantitative PCR. Copy number changes may be detected, for example, with the Illumina Infinium II whole genome genotyping assay or Agilent Human Genome CGH Microarray (Steemers et al., 2006).

Nucleic acid modifications can be assayed by methods described in, e.g., US Patent No. 7,186,512 and patent publication WO/2003/023065. Methylation profiles may be determined, for example, by Illumina DNA Methylation OMA003 Cancer Panel.

SNPs and mutations can be detected by hybridization with allele-specific probes, enzymatic mutation detection, chemical cleavage of mismatched heteroduplex (Cotton et al., 1988), ribonuclease cleavage of mismatched bases (Myers et al., 1985), mass spectrometry (US Patent Nos. 6,994,960, 7,074,563, and 7,198,893), nucleic acid sequencing, single strand conformation polymorphism (SSCP) (Orita et al., 1989), denaturing gradient gel electrophoresis (DGGE) (Fischer and Lerman, 1979a; Fischer and Lerman, 1979b), temperature gradient gel electrophoresis (TGGE) (Fischer and Lerman, 1979a; Fischer and Lerman, 1979b), restriction fragment length polymorphisms (RFLP) (Kan and Dozy, 1978a; Kan and Dozy, 1978b), oligonucleotide ligation assay (OLA), allele-specific PCR (ASPCR) (US Patent No. 5,639,611), ligation chain reaction (LCR) and its variants (Abravaya et al., 1995; Landegren et al., 1988; Nakazawa et al., 1994), flow-cytometric heteroduplex analysis (WO/2006/113590) and combinations/modifications thereof.

In one embodiment, the detection of mutations is carried out by using a restriction enzyme which only digests one variant of the biomarker but not other variants of the biomarker. As is know in the art, restriction enzymes faithfully recognize particular stretches of polynucleotides and the change of one or more nucleotides within the stretch of polynucleotides will mostly likely make the polynucleotide unrecognizable and indigestible by the enzyme. As such, the detection of one variant of a biomarker may be aided by digesting away some or all of the other variants that can be recognized by the enzyme. The variant to be detected can be a wild-type variant or a mutant variant.

Gene expression levels may be determined by the serial analysis of gene expression (SAGE) technique (Velculescu et al., 1995), quantitative PCR, quantitative reverse transcription PCR, microarray analysis, and next generation DNA sequencing, as known in the art.

In general, the methods for analyzing genetic aberrations are reported in numerous publications, not limited to those cited herein, and are available to skilled practitioners. The appropriate method of analysis will depend upon the specific goals of the analysis, the condition/history of the patient, and the specific cancer(s), diseases or other medical conditions to be detected, monitored or treated.

### Biomarkers associated with diseases or other medical conditions

Many biomarkers may be associated with the presence or absence of a disease or other medical condition in a subject. Therefore, detection of the presence or absence of such biomarkers in a nucleic acid extraction from isolated microvesicles, according to the methods disclosed herein, may aid diagnosis, prognosis, or monitoring the progress or reoccurrence of the disease or other medical condition in the subject.

For example, TMPRSS2:ERG is a fusion gene between trasmembrane protease serine 2 (TMPRSS2) and v-ets erythroblastosis virus E26 oncogene homolog (ERG) and is present in 40-80% of positive prostate cancer biopsies. As described in WO 2009/100029, detection of the presence or absence of the TMPRSS2:ERG fusion gene in nucleic acids extracted from microvesicles isolated from a patient's urine sample may aid in the diagnosis of prostate cancer in the patient. For another example, the human ERG gene, i.e., *Homo sapiens* v-ets erythroblastosis virus E26 oncogene homolog (avian), is a biomarker for prostate cancer. A higher ERG expression in post-DRE urine was found to be associated with the diagnosis of prostate cancer on biopsy (Rice et al., 2010).

Many biomarkers have also been found to influence therapy selection for a particular patient. The detection of the presence or absence of such biomarkers in a nucleic acid extraction from isolated microvesicles, according to the methods disclosed herein, may aid in therapy selection in a given patient. For example, the SLC45A3:BRAF fusion event occur in 1-2% of prostate cancers and its presence in prostate cells can induce a neoplastic phenotype that was sensitive to RAF and mitogen-activated protein kinase kinase (MAPK2K1) inhibitors (Palanisamy et al., 2010). The identification of biomarkers such as the SLC45A3:BRAF fusion gene expression in nucleic acids extracted from isolated particles from a patient's urine sample can guide the skilled practitioner in the selection of treatment for the patient.

Selection of an individual from whom the microvesicles are isolated is performed by the skilled practitioner based upon analysis of one or more of a variety of factors. Such factors for consideration are whether the subject has a family history of a specific disease (e.g., a cancer), has a genetic predisposition for such a disease, has an increased risk for such a disease, has physical symptoms which indicate a predisposition, or environmental reasons. Environmental reasons include lifestyle, exposure to agents which cause or contribute to the disease such as in the air, land, water or diet. Other reasons to select an individual for performing the methods disclosed herein include previous history with the disease, being currently diagnosed with the disease prior to therapy or after therapy, being currently treated for the disease (undergoing therapy), or being in remission or recovery from the disease.

### Example 1: Materials and Methods

A biomarker analysis for prostate cancer in urine-derived microvesicles was performed by obtaining urine samples, isolating microvesicles from the samples, extracting nucleic acids from the microvesicles, and detecting the expression levels of ERG, TMPRSS2:ERG, PSA, and GAPDH genes.

20 ml spot urine samples were obtained from five groups of individuals. These urine samples were voided urine samples that were obtained without a digital rectal exam (DRE) or a prostatic massage prior to urine collection. The five groups and the number of individuals in each group are: the control group (Control, males under 35 years old) with 40 individuals; the group characterized by a biopsy positive for prostate cancer (Bx Pos) with 38 individuals, the group characterized by a biopsy negative group (Bx Neg) with 39 individuals, the group characterized by radical prostatectomy no evidence of disease group (RP NED) with 35 individuals, and the group characterized by patients without a biopsy group or a diagnosis (No Bx) with 45 individuals.

The individuals' age between the five groups was compared. As shown in Figure 1, individuals in the Control group are significantly younger than those in each of the other four groups. The urine samples from the individuals in the five groups were stored at 4°C before further processing.

To extract nucleic acids from the urine samples, the urine samples were filtered through 0.8 µm filters (Nalgene). The filtrate was then centrifuged at 20,000g for 1 hour at 4°C in an angle head rotor. The supernatant was removed and discarded. Alternatively, the filtrate is processed through a 100 kDa molecular weight filter (cellulose nitrate membrane filter unit, Nalgene) to concentrate the filtrate to 100-200 ul. Prior to RNA extraction, the samples are incubated with RNase inhibitors. Then, the urine microvesicles were lysed in RLT buffer (Qiagen) plus 10 µl/ml betamercaptoethanol and processed using the Qiagen RNeasy Plus kit. The ribonucleic acids were eluted in 16 µl nuclease-free water.

The profile of the extracted nucleic acids was analyzed using an Agilent Bioanalyzer, and peaks corresponding to 18S and 28S rRNAs were detected. 12 µl of the extracted RNA were reverse transcribed into cDNA using Superscript VILO cDNA Synthesis Kit (Invitrogen 11754-050). The reverse transcription reaction mixture was made according to the following scheme (Table 1). The "5X" or "10X" indicates that the original concentration is 5 times or 10 times the final concentration in the reaction mixture, respectively. The unit "µl" is a short-hand for microliter.

**Table 1. Reverse transcription reaction mixture scheme for each reverse transcription reaction.**

| **Original reagent** | **Amount (µl)** |
|---|---|
| 5X VILO^{™} Reaction Mix | 4 |
| 10X SuperScript^{®} Enzyme | 2 |
| Mix | |
| RNA (up to 2.5 µg) | 12 |
| Nuclease free water | 2 |
| Total volume | 20 |

The reverse transcription was performed in a Veriti PCR machine (Applied BioSystems) under the following conditions: 25°C for 10 min, 42°C for 70 min, 85°C for 5 min, hold at 4°C before storing the reaction at -20°C.

Then, 1µl of the resulting cDNA product was used as template to perform Real-time PCR. The primers and probes used for RT-PCR were commercially obtained from Life Technologies^{™}, as follows: human Androgen Receptor (part number Hs00907244_m1); human BIRC5 (surviving, part number Hs00153353_m1); human GAPDH (part number 4326317E- 1009037); human PSA (part number Hs03083374_m1); human NCOA2 (part number Hs00197990_m1); human PCA3(part number Hs01371938_m1); human RAD21 (part number Hs00366726_m1); human TMPRSS2 (part number Hs00237175_m1); human ERG gene (part number Hs01554635_m1); and human TMPRSS2:ERG (here abbreviated as "T:E") gene (part number Hs03063375_m1). The human ERG gene primers and probe (part number Hs01554635_M1) detect four variants of the human ERG gene, i.e., variants 1-4. The real time-PCR experiments were repeated with each gene four times for each sample. The expression levels were represented with Ct (Cycle Threshold) values. Ct is a relative measurement of gene concentration in a PCR reaction as is known to persons skilled in the art. The average Ct values, per patient sample, were obtained for each gene in the samples.

The real time PCR results for each of the samples were analyzed using the RQ method with the DataAssist^{™} Program (obtained from Applied BioSystems). RQ analysis requires a designated reference gene whose expression level is constant across all test samples and whose expression is not affected by the experimental treatment under study (Wong and Medrano, 2005). A reference gene may be GAPDH, PSA or PCA3. In addition, RQ analysis may require a calibrator sample upon which relative expression of a target gene in the test sample can be determined (Wong and Medrano, 2005). A calibrator group may be the Control group or the Bx Neg group. The difference of the expression levels between the groups was measured statistically by calculating the P values as is known to a person skilled in the art. In some cases, a P value smaller than, e.g., 0.01 was deemed to be statistically significant.

### Example 2: Comparison of the relative expression level of ERG and various reference nucleic acids

Different combinations of the reference genes (GAPDH and PSA) and the calibrator group (Control and Bx Neg) were used for the RQ analysis of human ERG gene expression.

In the first combination, GAPDH was used as the reference gene, and the Control groups were used as the calibrator group. As shown in Figure 2, the expression levels of ERG in urine microvesicles from the Bx Pos, Bx Neg, and No Bx group were about 10.3, 2.3, and 1.1 times the expression levels of ERG in the Control group, respectively. The P values of the Bx Pos group versus the Control, Bx Neg, and No Bx groups were 0.0017, 0.0093, and 0.002, respectively. The difference of ERG expression levels between Bx Pos and Control, Bx Pos and Bx Neg, and Bx Pos and No Bx, were statistically significant because all numbers (0.0017, 0.0093, and 0.002, respectively) were smaller than 0.01.

In the second combination, GAPDH was used as the reference gene, and the Bx Neg groups were used as the calibrator group. As shown in Figure 3, the expression levels of ERG in urine microvesicles from the Bx Pos group were about 4.5 times the expression levels of ERG in the Bx Neg group. This difference is statistically significant (P=0.0064). The expression levels in the Control and No Bx groups were about 0.4 and 0.5 times the level in the Bx Neg group, respectively.

In the third combination, the PSA gene was used as the reference gene, and the Control group was used as the calibrator group. As shown in Figure 4, the expression levels of ERG in urine microvesicles from the Bx Pos, Bx Neg and No Bx group were about 9.2, 1.1 and 3.4 times the expression level of ERG in the Control group, respectively. The P values of the Bx Pos group versus the Control, Bx Neg, and No Bx groups were 0.0022, 0.0027, and 0.0551, respectively. The difference of ERG expression levels between Bx Pos and Control, as well as Bx Pos and Bx Neg, were statistically significant because all numbers (0.0022 and 0.0027 respectively) were smaller than 0.01. In contrast, the difference of ERG expression levels between Bx Pos and No Bx was more likely not significant because the number 0.0551 was bigger than 0.01.

In the fourth combination, the PSA gene was used as the reference gene, and the Bx Neg group was used as the calibrator group. As shown in Figure 5, the expression levels of ERG in urine microvesicles from the Bx Pos group were about 8.3 times the expression level of ERG in the Bx Neg group. This difference is statistically significant (P=0.0025). The expression levels in the Control and No Bx groups were about 0.9 and 3.0 times of the level in the Bx Neg group, respectively.

The ERG expression analysis in each of the four combinations demonstrates that ERG expression level is significantly higher in patients with a designation of positive prostate cancer biopsy than the level in other patient groups. Therefore, the method of using urine microvesicles can be used, as disclosed herein, to detect prostate cancer biomarkers including, e.g., ERG gene expression level. In this method, prostate tissue biopsy may be bypassed. Furthermore, no digital rectal exam or prostatic massage is required prior to urine collection.

### Example 3: Comparison of the relative expression levels of various biomarkers and reference nucleic acids

Based on the RT PCR results, RQ analysis was further performed on all the ten genes: androgen receptor (AR), BIRC5 (survivin), ERG, GAPDH, KLK3 (PSA), NCOA2, PCA3, RAD21, TMPRSS2:ERG, and TMPRSS2.

In one occasion, GAPDH was used as the reference gene and Bx Pos was used as the calibrator group. As shown in Figure 13, the expression of the ten genes varied in Bx Pos and Bx Neg groups. The RQ value for each gene in the five groups was calculated, and P value for between Bx Pos and each of the other four groups was also obtained. As shown in Table 2, the average RQ value varied and the P value also varied for different genes in different groups. For example, between Bx Pos and Bx Neg groups, the expression level difference for the ten genes was not statistically significant except for ERG (P=0.0093).

**Table 2. Differential expression of genes in the five patient groups (GAPDH as the reference gene).**

| Assay | BxNe g (RQ) | BxNeg (P-Value) | BxPos (RQ) | BxPos (P-Value) | Cont (RQ) | Cont (P-Value) | No Bx (RQ) | No Bx (P-Value) | RP NED (RQ) | RPNED (P-Value) |
|---|---|---|---|---|---|---|---|---|---|---|
| AR | 1.6708 | 0.1962 | 1 | 1 | 0.8721 | 0.4253 | 1.2028 | 0.3993 | 1.27 | 0.754 |
| BIRC5 | 0.5868 | 0.0866 | 1 | 1 | 0.5836 | 0.0679 | 0.5249 | 0.0289 | 0.9102 | 0.7328 |
| ERG | 0.2253 | 0.0093 | 1 | 1 | 0.0998 | 0.0017 | 0.1125 | 0.002 | 0.207 | 0.0082 |
| GAPDH | 1 | NaN | 1 | NaN | 1 | NaN | 1 | NaN | 1 | NaN |
| PSA | 1.3021 | 0.1314 | 1 | 1 | 0.7327 | 0.1186 | 0.8528 | 0.4108 | 0.0222 | 0 |
| NCOA2 | 0.7692 | 0.2689 | 1 | 1 | 1.7034 | 0.0218 | 0.8252 | 0.4123 | 1.4822 | 0.3613 |
| PCA3 | 0.3844 | 0.023 | 1 | 1 | 0.1102 | 0.001 | 0.3102 | 0.0106 | 0.0279 | 4.00E-04 |
| RAD21 | 0.6895 | 0.1063 | 1 | 1 | 1.0769 | 0.7331 | 0.6113 | 0.0437 | 0.9647 | 0.9312 |
| T:E | 0.3832 | 0.1441 | 1 | 1 | 0.0083 | 0.0014 | 0.1086 | 0.0037 | | |
| IMP | 0.9909 | 0.9462 | 1 | 1 | 1.3922 | 0.0379 | 0.8621 | 0.29 | 1.1782 | 0.7806 |

In another occasion, PSA was used as the reference gene, and Bx Pos was used as the calibrator group. As shown in Figure 14, the expression of the ten genes varied in Bx Pos and Bx Neg groups. The RQ value for each gene in the five groups was calculated, and P value for between Bx Pos and each of the other four groups was also obtained. As shown in Table 3, the average RQ value varied and the P value also varied for different genes in different groups. For example, between Bx Pos and Bx Neg groups, for BIRC5 (Survivin), ERG, and PCA3 genes the expression level difference was statistically significant (P = 0.0033, 0.0027, and 0.0042, respectively). But for other seven genes, the difference was not statistically significant.

**Table 3. Differential expression of genes in the five patient groups (PSA as the reference gene)**

| Assay | BxNeg (RQ) | BxNeg (P-Value) | BxPos (RQ) | BxPos (P-Value) | Cont (RQ) | Cont (P-Value) | No Bx (RQ) | No Bx (P-Value) | RP NED (RQ) | RP NED (P-Value) |
|---|---|---|---|---|---|---|---|---|---|---|
| AR | 1.1545 | 0.523 | 1 | 1 | 2.1322 | 0.1486 | 2.3935 | 0.1282 | 391.7561 | 0.0718 |
| BIRC5 | 0.3561 | 0.0033 | 1 | 1 | 0.7264 | 0.2872 | 1.4271 | 0.474 | 529.8685 | 0.1514 |
| ERG | 0.1194 | 0.0027 | 1 | 1 | 0.1071 | 0.0022 | 0.3639 | 0.0551 | 58.6867 | 0.1779 |
| GAPDH | 0.4574 | 0.0724 | 1 | 1 | 0.9556 | 0.8938 | 1.3112 | 0.4776 | 196.5095 | 0.0567 |
| PSA | 1 | NaN | 1 | NaN | 1 | NaN | 1 | NaN | 1 | NaN |
| NCOA2 | 0.5809 | 0.1242 | 1 | 1 | 2.366 | 1.24E-02 | 2.5811 | 0.1137 | 274.2028 | 0.0064 |
| PCA3 | 0.3872 | 0.0042 | 1 | 1 | 0.2057 | 2.00E-04 | 0.4063 | 0.0048 | 1.3974 | 0.1783 |
| RAD21 | 0.305 | 0.0511 | 1 | 1 | 0.9596 | 0.9154 | 1.0017 | 0.9972 | 158.7825 | 0.0234 |
| T:E | 0.2027 | 0.0105 | 1 | 1 | 0.0249 | 8.00E-04 | 0.1596 | 0.0037 | | |
| TMP | 0.6138 | 0.0188 | 1 | 1 | 1.8473 | 0.0121 | 1.8907 | 0.1445 | 242.1427 | 0.0263 |

### Example 4: Normalization of the relative expression levels of various biomarkers and GAPDH as a reference nucleic acid

In addition, based on the Ct values for each gene in the samples as shown above, the gene expression levels was calculated using the formula represented by 2^{(-ΔCt)} using GAPDH as the reference gene. This normalization can be done with any reference gene as shown in the following example.

### Example 5: Normalization of the relative expression levels of various biomarkers and GAPDH and/or PSA as a reference nucleic acid

In addition to the ERG levels calculated using GAPDH as the reference gene, the ERG expression level in each sample was calculated using PSA as the reference gene through
2^{(-ΔCt)}. Specifically, the expression levels were calculated using the Ct values for the ERG, GAPDH and PSA in each sample as follows:
When GAPDH is used as the reference gene, ΔCt=Ct_{ERG}-Ct_{GAPDH} as in Figure 6.

When PSA is used as the reference gene, ΔCt=Ct_{ERG}-Ct_{PSA} as in Figure 7.

The ERG expression level=2^{(-ΔCt)}. The values thus obtained are used for the graphs (Y axis) in Figures 6 and 7.

As shown in both Figures 6 and 7, ERG expression levels are generally highest in the Bx Pos group compared to the expression levels in other groups. There are some outliners such as the one with a value of about 0.01 in the Bx Neg group in Figure 6 and the one with a value of about 0.0275 in the No Bx group in Figure 7. The outliners may represent disease-positive patients missed by conventional biopsy diagnostics. The detection of these outliners suggests that the method disclosed herein may be more sensitive than current diagnostic methods for identifying patients who need further biopsy analysis.

### Example 6: Comparison of the expression levels of ERG fusion nucleic acid and a variety of reference nucleic acids

Different combinations of the reference gene (GAPDH and PSA) and the Control group as the calibrator group were used for the RQ analysis of human TMPRSS2:ERG fusion gene expression.

In the first combination, GAPDH was used as the reference gene, and the Control groups were used as the calibrator group. As shown in Figure 8, the expression levels of TMPRSS2:ERG fusion gene in urine microvesicles from the Bx Pos, Bx Neg, and No Bx groups group were about 125.9, 46.4, and 13.1 times the expression level of TMPRSS2:ERG fusion gene in the Control group, respectively. The P values of the expression levels in Bx Pros versus the Control, Bx Neg, and No Bx were 0.0014, 0.1441, and 0.0037. The difference of TMPRSS2:ERG expression levels between Bx Pos and Control groups, as well as between Bx Pos and No Bx groups, were statistically significant because both numbers (0.0014 and 0.0037) were smaller than 0.01. In contrast, the difference between Bx Pos and Bx Neg was not significant because the number (0.1441) is bigger than 0.01.

In the second combination, PSA was used as the reference gene, and the Control groups were used as the calibrator group. As shown in Figure 9, the expression levels of TMPRSS2:ERG fusion gene in urine microvesicles from the Bx Pos, Bx Neg, and No Bx groups were about 39.5, 8.1 and 6.4 times the expression level of TMPRSS2:ERG fusion gene in Control group, respectively. The P values of the expression levels in Bx Pros versus the Control, Bx Neg, and No Bx were 0.0008, 0.0105, and 0.0037, respectively. The difference of TMPRSS2:ERG expression levels between Bx Pos and Control groups, as well as between Bx Pos and No Bx groups, were statistically significant because both numbers (0.0008 and 0.0037) were smaller than 0.01. In contrast, the difference between Bx Pos and Bx Neg was more likely not significant because the number (0.0105) is bigger than 0.01.

In the third combination, GAPDH was used as the reference gene, and the Bx Neg was used as the calibrator group. As shown in Figure 10, the expression levels of TMPRSS2:ERG fusion gene in urine microvesicles from the Bx Pros group were about 2.7 times the expression level of TMPRSS2:ERG fusion gene in Bx Neg group. And this difference is statistically not significant (P=0.1171). The expression levels in the Control and No Bx groups were about 0.02 and 0.28 times of the level in the Bx Neg group, respectively.

The TMPRSS2:ERG fusion gene expression analysis described above demonstrates that the TMPRSS2:ERG fusion gene expression level is significantly higher in patients with a designation of positive prostate cancer biopsy than the level in the Control groups. However, the expression of TMPRSS2:ERG fusion gene in Bx Pos is more likely not significantly different from that in the Bx Neg group.

Therefore, the method of using urine microvesicles disclosed herein can detect prostate cancer biomarker of TMPRSS2:ERG fusion gene expression level. However, the expression levels of TMPRSS2:ERG fusion gene is less sensitive than the expression of ERG in distinguishing the Bx Pos group from the Bx Neg group.

### Example 7: Comparison of the relative expression levels of ERG, ERG fusion nucleic acid and a reference nucleic acid.

Furthermore, ERG and TMPRSS2:ERG gene expression analysis in the Bx Pos and Bx Neg groups was performed based on delta CT values
(ΔCt=Ct_{target}-Ct_{reference}) derived from the Ct values as detailed above. The reference gene was GAPDH.

As shown in Figure 15, the delta Ct values of ERG gene was plotted for each individual in the Bx Pos and Bx Neg groups. There were about 86.05% of samples with detectable signals. The Wilcoxon P value between these two groups was 0.00136, suggesting that the difference was statistically significant.

As shown in Figure 16, the delta Ct values of TMPRSS2:ERG gene was plotted for each individual in the Bx Pos and Bx Neg groups. There were about 54.65% of sample with dateable signals. The Wilcoxon P value between these two groups was 0.07089, suggesting that the difference was not statistically significant in these two particular patient populations.

These delta Ct analysis results suggest that the ERG expression is more sensitive that TMPRSS2:ERG to distinguish the two patient groups, i.e., Bx Pos and Bx Neg using the method disclosed herein. Therefore, ERG expression may be a better biomarker than TMPRSS2:ERG for differentiating the Bx Pos group from the Bx Neg group.

### Example 8: Detection of SLC45A3:BRAF fusion nucleic acid in urine sample

RNA was extracted from the urine sample of the subject CaP66 (a BxPos patient) with the same method as detailed above. In particular, neither DRE nor cellular pellets were carried out in the RNA extraction process. The extracted RNA was used for human SLC45A3:BRAF fusion gene and TMPRSS2:ERG fusion gene analysis by quantitative PCR in the same procedure as detailed above. The primers used for PCR were: SLC45A3-BRAF fusion forward: CTGCACGCGCTGGCTC (SEQ ID NO: 1); SLC45A3-BRAF fusion reverse: TCTTCATCTGCTGGTCGGAA (SEQ ID NO: 2). The probe was SLC45A3-BRAF probe: CAAATTCTCACCAGTCCGTCT (SEQ ID NO: 3).

The PCR results were depicted as amplification plots. As shown in Figure 11, the expression of human SLC45A3:BRAF fusion gene could be clearly detected. The Ct value for SLC45A3:BRAF was about 31 when the threshold line was set at 0.015. As shown in Figure 12, the expression of human TMPRSS2-ERG could also be readily detected in the same sample. The Ct value for TMPRSS2-ERG was about 23.5 when the threshold line was set at 0.12.

Therefore, the noninvasive method disclosed herein was able to detect the rare fusion event between SLC45A3 and BRAF. The rare fusion event was previously detected in a biopsy tissue (Palanisamy et al., 2010). In this new method, cells were removed from the urine sample without DRE-like procedures and nucleic acids were extracted from microvesicles isolated from the urine sample. This is the first time that this rare mutation has been detected in a noninvasive manner.

### Example 9: Biomarkers for prostate cancer recurrence

Currently, prostate cancer recurrence is assessed based on serum PSA protein levels. This serum PSA method cannot tell whether the recurrence is a local recurrence in the prostate gland or a systemic cancer metastasis. However, patients, who show both PSA gene expression and elevated serum PSA protein levels, are usually predicted to more likely have a local recurrence than systematic metastasis. Such knowledge can guide treatment plans because local recurrence should usually be treated with localized radiation therapy while systematic metastasis should usually be treated with a systemic therapy such as chemotherapy. Such knowledge may help guide adjuvant therapy.

Using the noninvasive method disclosed herein, the studies described herein demonstrate that the expression of prostate biomarker genes such as PSA could be detected in the urine samples from patients who had undergone Ablation therapy or Radical Prostatectomy. The detection of such markers may be used, in combination with serum PSA protein levels, to assess the likelihood of local recurrence of prostate cancer.

In this exemplary embodiment, urine samples were obtained from five groups of patients: the Control group with healthy individuals (Cont), the group of patients who had undergone ablation therapy but exhibited no evidence of disease (ABL NED), the group of patients who had undergone radical prostatectomy but exhibited no evidence of disease (RP-NED), the group of patients who had undergone ablation therapy and were alive with disease (ABL AWD), and the group of patients who had undergone radical prostatectomy and were alive with disease (RP-AWD).

RNA from each urine sample was extracted using the same method as disclosed above without DRE or cellular pellet collections. Similarly, quantitative PCR was performed to measure the expression of PSA and GAPDH genes in the samples. The Ct values for PSA and GAPDH were used to derive the PSA expression levels (calculated as 2^{-ΔCt}, ΔCt=Ct_{PSA}-Ct_{GAPDH}).

As shown in Figure 17, PSA expression in the urine following RP or Ablation therapy can be detected. For example, in two samples in the ABL AWD group, the levels were around 0.5. Similar analysis can be applied for other prostate genes such as PCA3.

### Example 10: Detection of Survivin in urine microvesicles

Prostate cancer growth is sometimes dependent on androgen receptor signaling triggered by 5α-dihydrotestosterone (DHT). Localized prostate cancer may be cured with prostatectomy and/or radiotherapy, but prostate cancer recurs in about 20-30% of patients. Androgen deprivation therapy is sometimes used to treat recurrent prostate cancer. In castration resistant prostate cancer (CRPC), the androgen receptor signaling is activated even though there are extremely low levels of androgens in patients treated with androgen deprivation therapy. The median survival time for these CRPC or hormone resistant (HR) patients are about 12.2 to about 21.7 months.

Currently, there are numerous new agents, e.g., abiraterone, MDV3100, sipuleucel-T (Provenge), cabazitaxel, for the treatment of patients with castration resistant prostate cancer (CRPC). However, there is no clinical guideline for treatment selections for different patients. It was previously found that microvesicles, including exosomes, carried high integrity RNA from their parent cells and could be used to reliably interrogate the transcriptional profile of various organs in a non-invasive manner. By microvesicle nucleic acid analysis, the studies described herein show a method for differentiating prostate cancer patients by examining the expression of Survivin gene in urine microvesicles. Survivin is an inhibitor of apoptosis and may be the underlying reason for hormone independent tumor growth (Zhang et al., 2005).

It was found that Survivin gene was differentially expressed in urine microvesicles from patients with advanced prostate cancer at different stages. Urine samples from patients were collected upon obtaining IRB approval and informed consent. The urine samples were collected prior to a digital rectal exam from four groups of patients: 1) radical prostatectomy with no evidence of disease (RP-NED, there were 37 patients in this group, n=37); 2) radical prostatectomy alive with disease (RP-AWD, n=22); 3) ablative therapy (external radiation therapy or cryotherapy) with no evidence of disease (ABL-NED, n=13); and 4) ablative therapy (external radiation therapy or cryotherapy) alive with disease (ABL-AWD, n=14). The RP-AWD patients were further stratified into HR (n=11) and non-HR (n=11) groups. Similarly, the ABL-AWD patients were stratified into HR (n=8) and non-HR (n=6) groups.

Urinary exosomal mRNA was isolated. GAPDH and Survivin were analyzed via RT-qPCR using the isolated mRNA. To compare expression levels, genes were standardized to GAPDH and relative quantitation (RQ) was calculated using the DataAssist program (v.2.0). Survivin gene expression levels were significantly higher in the RP-AWD group compared to the RP-NED group (RQ=3.63, p=0.03). A similar result was observed for patients with ablation therapy as their primary treatment in that Survivin gene expression levels were higher in ABL-AWD group compared to the ABL-NED group (RQ=3.23, p=0.057). These data indicate that Survivin expression is generally higher in patients who are alive with disease that patients without evidence of disease no matter they underwent radical prostatectomy or ablative therapy.

The expression level of Survivin in urine microvesicles can also differentiate HR patients and non-HR patients. Increased Survivin expression levels were associated with castration resistance for RP-NED versus RP-AWD HR (RQ=5.49, p=0.0422); and ABL-NED versus ABL-AWD HR (RQ=5.58, P=0.0291). Additionally, Survivin expression levels were significantly higher in the ABL-AWD HR patients versus ABL-AWD non-HR patients (RQ=6.30, p=0.027).

In contrast, based on the data generated in this example, Survivin expression level alone is likely unable to differentiate RP NED versus RP AWD non-HR (RQ=1.78, P=0.4433), RP AWD non-HR versus RP AWD HR (RQ=3.07, P=0.1043), and ABL NED versus ABL AWD non-HR patients (RQ=0.89, P=0.794).

Survivin expression levels were elevated in urinary microvesicles from patients with HR compared to patients without HR in some prostate cancer patient population. This non-invasive Survivin assay method, and derivations based upon it, may be utilized to follow prostate cancer patients over time for purposes of monitoring disease progression, to assay efficacy in response to treatment, or to select treatment plans. The assay and derivatives of it may additionally be used in support of drug discovery and biomarker discovery.

### Example 11: Diagnosis of prostate cancer by detection of biomarkers in urinary microvesicles as compared to detection of biomarkers in prostate biopsy

The above Examples 1-9 have shown that urinary microvesicles contain prostate specific biomarkers, e.g., prostate specific mRNA transcripts. Here, these studies demonstrate that the analysis of biomarkers in urinary microvesicles can substitute the analysis of biomarkers in prostate biopsy tissues. For instance, the detection of TMPRSS2:ERG (T:E) expression in urinary microvesicles is consistent with the expression of TMPRSS2:ERG (T:E) in prostatectomy tissue.

Spot or random urine samples were collected without prostate massage from 163 men with Columbia University IRB approval. These 163 men were stratified into four groups: transrectal ultra sound (TRUS) prostate biopsy negative (Bx Neg, n=39), TRUS biopsy positive (Bx Pos, n=47), post-radical prostatectomy (RP, n=37) and controls (males <35 yrs, n=40). All groups except the control (males <35 yrs) were age matched. Additionally, whole-mount paraffin embedded prostate sections were obtained from 11 patients who underwent RP and had pre-RP urine specimens available. Urine samples were stored at 4°C and 0.8 µm filtration was used to remove whole cells and debris. Urinary microvesicular RNA and tissue RNA were isolated and analyzed using RT-qPCR according to a procedure similar to that described in Example 1. The primers and probes used for RT-PCR were commercially obtained from Life Technologies^{™}, including human TMPRSS2:ERG (here abbreviated as "T:E") gene (part number Hs03063375_m1).

As a result, it was found that mean serum PSA levels were similar in the Bx Neg and Bx Pos groups. In addition, it was found that T:E fusion events occurred in 68% of Bx Pos patients, 44% of Bx Neg patients, 5% of controls, and no patient of the post RP group. The detection rate of the T:E fusion in the Bx Pos group using microvesicular RNA extracted from urine samples was consistent with previous reports where T:E fusion events were detected using RNA extracted from biopsy tissue samples. The detection of T:E fusion events in the urine samples from biopsy negative patients may be due to false negative biopsy results or early detection of T:E expression in premalignant lesions. Furthermore, it was found that T:E expression was lost in the 4 Bx Pos patients who underwent prostatectomy and were retested after surgery.

The studies described herein demonstrate that the T:E expression analysis using urine samples can replace the T:E analysis using prostate biopsy tissue samples because the results are consistent in paired urine and biopsy tissue samples. Paired pre-radical prostatectomy urine samples and prostate biopsy tissue samples were collected from 11 patients. The prostate biopsy tissue samples include tissue samples from benign regions that surround the cancer regions as well as tissue samples from the cancer regions. The tissue samples were processed to obtain tissue sections that can be used for pathological examinations according to standard protocols.

Microvesicles in the pre-RP urine samples were isolated, RNA from the isolated microvesicles was extracted, and the expression of T:E was examined. The microvesicles were isolated using filtration concentrator method. As shown in Table 4, 7 patients with T:E positive (CaP1, CaP6, CaP63, CaP99, CaP108, CaP231, and CaP232) and 4 patients with T:E negative (CaP4, CaP7, CaP77, and CaP124) were found.

T:E expression was also examined in the corresponding prostate biopsy tissue sections in the same group of 11 patients. As shown in Table 4, 8 patients with T:E positive (CaP1, CaP6, CaP63, CaP99, CaP7, CaP108, CaP231, and CaP232) and 3 patients with T:E negative (CaP4, CaP77, and CaP124) were found by analyzing T:E expression in the prostate tissue sections from the cancer regions. A comparison of the T:E expression analysis between pre-RP urine samples and prostate tissue section indicate that in 10 of 11 (91%) patients the two methods gave rise to the same result, and that the sensitivity and specificity of T:E detection using urine samples were 89% and 100%, respectively.

T:E expression in sections from biopsy benign regions was also examined. As shown in Table 4, T:E expression in sections from benign region was positive in 5 of the 11 patients. The positive expression is possibly associated with high grade prostatic intraepithelial neoplasia (PIN) (HGPIN) in these regions (Furusato et al., 2008).

Tumor heterogeneity in the expression of T:E was also noted when multiple cancer foci were independently examined at the tissue level. For example, in one Bx Pos patient (CaP7), T:E expression was negative in one tumor sample (the biopsy tissue sections B1, B2, B6, B7, and B8) but was positive in the other tumor sample (the biopsy tissue sections B9, B10, B11, B12, and B13). This observation is consistent with previous findings seen in, e.g, (Furusato et al., 2008). The example demonstrates that the non-invasive urine microvesicle test that can be carried out without digital rectal exam or prostate massage prior to urine collection has a very high sensitivity and specificity for the detection of prostate specific markers such as T:E.

**Table 4. RT-qPCR results of T:E expression in urine and prostate biopsy tissue samples. ND - not detected after 40 cycles of PCR.**

| **Patient ID** | **T:E detection in urinary exosomes (yes/no)** | **T:E detection in biopsy (yes/no)** | **Urine exosome T:E (ct)** | **Biopsy sections analyzed** | **Slide tissue designation (tumor/ benign)** | **Biopsy T:E (ct)** |
|---|---|---|---|---|---|---|
| **CaP1** | yes | yes | 30.45 | C2, C6, C12, C17, C19 | Benign | 29.98 |
| | | | | C9, C10, C13, C15, C20 | Tumor | 27.82 |
| **CaP4** | no | no | NO | C15, C16, C17, C18, C20 | Benign | ND |
| | | | | C5, C6, C7, C8, C9 | Tumor | ND |
| | | | | C10, C11, C13, C14, C19 | Tumor | ND |
| **CaP6** | yes | yes | 28.29 | C5, C6, C7, C8, C12 | Benign | 27.41 |
| | | | | C14, C15, C16, C17 | Tumor | 32.88 |
| | | | | C2, C9, C10, C11, C13 | Tumor | 26.54 |
| **CaP63** | yes | yes | 31.24 | C6, C12, C15, C19, C22 | Benign | ND |
| | | | | C7, C10, C11, C17 | Tumor | 25.83 |
| **CaP99** | yes | yes | 32.92 | C11(3), C21 (2) | Benign | ND |
| | | | | C16, C17, C18, C19, C20 | Tumor | 28.58 |
| **CaP7** | no | yes | NO | B3, B4, B5, B19, B23 | Benign | ND |
| | | | | B1, B2, B6, B7, B8 | Tumor | ND |
| | | | | B9, B10, B11, B12, B13 | Tumor | 34.14 |
| **CaP77** | no | no | ND | C1, C2, C3, C4, C5 | Benign | ND |
| | | | | C8, C10, C11, C12, C13 | Tumor | ND |
| | | | | C14, C15, C16, C18, C20, C22 | Tumor | N D |
| **CaP108** | yes | yes | 24.84 | B1, B2, B3, B7, B21 | Benign | 31.57 |
| | | | | B10, B11, B12, B13,B14 | Tumor | 17.19 |
| | | | | B15, B16, B17, B18 | Tumor | 19.77 |
| **CaP124** | no | no | ND | C1, C3, C4, C10, C15 | Benign | N D |
| | | | | C7, C8, C9, C11 | Tumor | N D |
| | | | | C12, C13, C14,C16 | Tumor | N D |
| **CaP231** | yes | yes | 24.66 | D2, D3, D4, D15 | Benign | 16.43 |
| | | | | D5, D6, D7, D8, D9 | Tumor | 15.07 |
| | | | | D10, D11, D12, D13, D14 | Tumor | N D |
| | | | | D16, D17, D18, 022 | Tumor | 18.38 |
| **CaP232** | yes | yes | 25.27 | C6, C7,C8, C9, C11 | Benign | 26.93 |
| | | | | C1, C2, C5, C10, C12 | Tumor | 17.73 |
| | | | | C13, C14, C15, C16, C18, C22 | Tumor | 16.61 |

These data demonstrate that the detection of biomarkers in urinary microvesicles is consistent with the detection of biomarkers in matched prostate tissue samples. The unique stability and yield of urinary microvesicle RNA as demonstrated in these studies hereby disclosed in this invention will likely broaden the role of microvesicles in future diagnostic testing and simplify sample handing without the variability and patient discomfort inherent to prostate massage.

### Example 12: Detection of additional prostate cancer biomarkers

Additional prostate cancer biomarkers from urine microvesicles were identified, and the studies described herein demonstrate that some of these have diagnostic values. The biomarkers and the reference genes include v-akt murine thymoma viral oncogene homolog 1 (AKT1), adenosylmethionine decarboxylase 1 (AMD1), annexin A3 (ANXA3), eukaryotic translation elongation factor 2 (EEF2), enhancer of zeste homolog 2 *(Drosophila)* (EZH2), glutathione S-transferase pi 1 (GSTP1), HFM1 ATP-dependent DNA helicase homolog *(S. cerevisiae)* (HFM1), beta- microseminoprotein (MSMB), nuclear receptor coactivator 2 (NCOA2), prostate transmembrane protein, androgen induced 1 (PMEPA1), prostate stem cell antigen (PSCA), olfactory receptor family 51 subfamily E member 2 (PSGR), RAD21 homolog *(S. pombe)* (RAD21), SMAD family member 4 (SMAD4), transglutaminase 4 (prostate) (TGM4), matrix metallopeptidase 9 *(gelatinase B, 92kDa gelatinase, 92kDa type IV collagenase)* (MMP9), glyceraldehyde-3-phosphate dehydrogenase (GAPDH) and kallikrein-related peptidase 3 (KLK3).

These biomarkers were examined using urine samples from biopsy positive and biopsy negative prostate cancer patients. Microvesicles were isolated from the urine samples, nucleic acids were extracted from the isolated microvesicles, and the expression levels of the biomarkers with the extracted nucleic acids were analyzed. The primers used for the expression analysis were from ABI (Applied BioSystems^{™}) and are listed in Table 5. With the expression data of these biomarkers, Receiver Operating Characteristic (ROC) curve analysis was performed.

**Table 5. List of genes examined in the urinary microvesicles**

| **Gene abbreviation** | **Gene Full Name** | **ABI catalogue number** |
|---|---|---|
| AKT1 | v-akt murine thymoma viral oncogene homolog 1 | Hs00178289_m1 |
| AMD1 | adenosylmethionine decarboxylase 1 | Hs00750876_s1 |
| ANXA3 | annexin A3 | Hs00974395_m1 |
| EEF2 | eukaryotic translation elongation factor 2 | Hs00157330_m1 |
| EZH2 | enhancer of zeste homolog 2 (Drosophila) | Hs01016789_m1 |
| GSTP1 | glutathione S-transferase pi 1 | Hs00168310_m1 |
| HFM1 | HFM1, ATP-dependent DNA helicase homolog (S. cerevisiae) | Hs01651101_m1 |
| MMP9 | matrix metallopeptidase 9 (gelatinase B, 92kDa gelatinase, 92kDa type IV collagenase) | Hs00234579_m1 |
| MSMB | microseminoprotein, beta- | Hs00738230_m1 |
| NCOA2 | nuclear receptor coactivator 2 | Hs00197990_m1 |
| PCA3 | prostate cancer antigen 3 (non-protein coding) | Hs01371938_m1 |
| PMEPA1 | prostate transmembrane protein, androgen induced 1 | Hs00375306_m1 |
| PSCA | prostate stem cell antigen | Hs00194665_m1 |
| PSGR | olfactory receptor, family 51, subfamily E, member 2 | Hs00951952_m1 |
| RAD21 | RAD21 homolog (S. pombe) | Hs00366726_m1 |
| TGM4 | transglutaminase 4 (prostate) | Hs00162710_m1 |
| GAPDH | glyceraldehyde-3-phosphate dehydrogenase | 4326317E-1009037 |
| KLK3 | kallikrein-related peptidase 3 | Hs03083374_m1 |
| SMAD4 | SMAD family member 4 | HS00929647_m1 |

ROC curve analysis is a method to determine the cutoff value for a clinical test. The ROC curve is a graph of sensitivity (y-axis) vs. specificity (x-axis). An important measure of the accuracy of the clinical test is the area under the ROC curve (AUC). If this area is equal to 1.0 then the ROC curve consists of two straight lines, one vertical from 0,0 to 0,1 and the next horizontal from 0,1 to 1,1. This test is 100% accurate because both the sensitivity and specificity are 1.0 so there are no false positives and no false negatives. On the other hand a test that cannot discriminate between normal and abnormal corresponds to an ROC curve that is the diagonal line from 0,0 to 1,1. The ROC area for this line is 0.5. ROC curve areas are typically between 0.5 and 1.0.

In a first analysis, GAPDH was used as the reference gene, and the expression of the biomarker genes EEF2, ANXA3, AKT1, and PMEPA1 was measured. The area under the curves from ROC analysis in comparing the biomarker expression levels between biopsy positive samples to biopsy negative samples was measured. As shown in Table 6, the AUC for the biomarker genes EEF2, ANXA3, AKT1, and PMEPA1 were between 0.70-0.77, suggesting that each of the above biomarkers individually can have diagnostic value. In contrast, the AUC for PSA gene was 0.31, suggesting that PSA may not have diagnostic value.

**Table 6. Measurement of area under the curves (AUC) for the tested biomarkers.**

| **Biomarker** | **AUC** |
|---|---|
| EEF2 | 0.77 |
| ANXA3 | 0.75 |
| AKT1 | 0.74 |
| PMEPA1 | 0.70 |
| PSA | 0.31 |

In a second analysis, KLK3 was used as the reference gene, and the expression of the biomarker genes AKT1, ANXA3, EEF2, EZH2, GSTP1, HFM1, MSMB, NCOA2, PMEPA1, PSCA, PSGR, RAD21, SMAD4, and TGM4 was measured.

The area under the curves from ROC analysis in comparing the biomarker expression levels between biopsy positive samples to biopsy negative samples was measured. As shown in Table 7 the AUC for the biomarker genes AKT1, ANXA3, EEF2, EZH2, GSTP1, HFM1, MSMB, NCOA2, PMEPA1, PSGR, RAD21, and SMAD4 were between 0.57-0.75, suggesting that each of the above biomarkers individually can have diagnostic value. In contrast, the AUC for PSCA and TGM4 were 0.50 and 0.47, respectively, suggesting that PSCA or TGM4 alone may not have diagnostic value.

**Table 7. ROC analysis of gene using KLK3 as the reference gene. Obs: number of patients examined for a particular marker gene in ROC analysis. Area: the area under the curve. Std. Err.: standard error in the statistical ROC analysis. 95% Conf. Interval: 95% confidence interval in the statistical ROC analysis.**

| **Gene** | **Obs** | **Area** | **Std. Err.** | **[95% Conf. Interval]** | |
|---|---|---|---|---|---|
| AKT1 | 46 | 0.7124 | 0.0760 | 0.56343 | 0.86133 |
| AMD1 | 23 | 0.7424 | 0.1196 | 0.50809 | 0.97676 |
| ANXA3 | 64 | 0.6903 | 0.0708 | 0.55158 | 0.82912 |
| EEF2 | 64 | 0.7510 | 0.0628 | 0.62805 | 0.87404 |
| EZH2 | 53 | 0.6710 | 0.0746 | 0.52483 | 0.81720 |
| GSTP1 | 46 | 0.6914 | 0.0795 | 0.53553 | 0.84733 |
| HFM1 | 15 | 0.6429 | 0.1594 | 0.33037 | 0.95534 |
| MSMB | 46 | 0.6419 | 0.0842 | 0.47695 | 0.80685 |
| NCOA2 | 84 | 0.6333 | 0.0618 | 0.51208 | 0.75451 |
| PMEPA1 | 46 | 0.6152 | 0.0852 | 0.44823 | 0.78225 |
| PSCA | 29 | 0.4952 | 0.1143 | 0.27118 | 0.71929 |
| PSGR | 18 | 0.5679 | 0.1445 | 0.28468 | 0.85112 |
| RAD21 | 84 | 0.6608 | 0.0596 | 0.54385 | 0.77766 |
| SMAD4 | 53 | 0.6232 | 0.0774 | 0.47146 | 0.77492 |
| TGM4 | 31 | 0.4667 | 0.1087 | 0.25353 | 0.67981 |

### Example 13: Detection of combination of biomarkers as a sensitive and specific diagnostic tool for prostate cancer

The studies described herein demonstrate here that a combination of biomarkers may sometimes yield more diagnostic value. For example, it was found that a combination of the biomarkers ERG and alpha-methylacyl-CoA racemase (AMACR) had a higher AUC value than either ERG or AMACR alone. Here, the expression of ERG and AMACR genes in 121 biopsy positive and biopsy negative patients was examined. The information of age, race, clinical Gleason, clinical stage, and the number of biopsy cores for these 121 patients are shown in Table 8

In a first analysis, KLK3 was used as a reference gene, the expression of ERG and AMACRwas measured, and ROC analysis for ERG alone, AMACR alone, and a combination of ERG and AMACR was performed.

**Table 8. Biopsy positive and negative patients in the study**

| | | **Biopsy Neg** | | **Biopsy Pos** | | **p-value** |
|---|---|---|---|---|---|---|
| | **N** | 49 | | 72 | | |
| | **Age** | 67 | | 66 | | 0.40 |
| **Race** | | | | | | 0.033 |
| | White | 34 | 81% | 38 | 58% | |
| | Black | 1 | 2% | 9 | 14% | |
| | Other | 7 | 17% | 19 | 28% | |
| | | | | | | 0.41 |
| | **PSA** | 6.0 | | 6.8 | | |
| **Clinical Gl eason** | | | | | | |
| | <7 | - | | 33 | 46% | |
| | 7 | - | | 29 | 40% | |
| | >7 | - | | 10 | 14% | |
| **Clinical Stage** | | | | | | |
| | T1c | - | 64 | 89% | | |
| | >T1c | - | 8 | 11% | | |
| **Number of Cores** | | 15.9 | 14 | | 0.11 | |

As shown in Figure 18, the AUC value for ERG alone was 0.7824 based on the expression of ERG in 94 patients. The standard deviation was 0.0468. The 95% confidence Internal was between 0.69058 and 0.87414. Among the 94 patients, there were 37 biopsy negative patients and 57 biopsy positive patients. The information of age, race, clinical Gleason, clinical stage, and the number of biopsy cores for these 94 patients are shown in Table 9

**Table 9. The 94 patients in ROC analysis of ERG alone**

| | | **Biopsy Neg** | | **Biopsy Pos** | | **p-value** |
|---|---|---|---|---|---|---|
| | **N** | 37 | | 57 | | |
| | **Age** | 68 | | 67 | | 0.41 |
| **Race** | | | | | | 0.033 |
| | White | 27 | 79% | 26 | 50% | |
| | Black | 1 | 3% | 8 | 15% | |
| | Other | 6 | 18% | 18 | 35% | |
| | **PSA** | 5.4 | | 6.6 | | 0.24 |
| **Clinical Gleason** | | | | | | |
| | <7 | - | | 27 | 47% | |
| | 7 | - | | 24 | 42% | |
| | >7 | - | | 6 | 11% | |
| **Clinical Stage** | | | | | | |
| | T1c | - | 49 | 87% | | |
| | >T1c | - | 8 | 13% | | |
| **Number of Cores** | | 15 | 14 | | 0.60 | |

As shown in Figure 19, the AUC value for AMACR alone was 0.7610 based on the expression of AMACR in 64 patients. The standard deviation was 0.0619. The 95% confidence Internal was between 0.63969 and 0.88236. Among the 64 patients, there were 25 biopsy negative patients and 39 biopsy positive patients. The information of age, race, clinical Gleason, clinical stage, and the number of biopsy cores for these 64 patients are shown in Table 10.

**Table 10. The 64 patients in the ROC analysis of AMACR alone**

| | | **Biopsy Neg** | | **Biopsy Pos** | | **p-value** |
|---|---|---|---|---|---|---|
| | **N** | 25 | | 39 | | |
| | **Age** | 67 | | 63 | | 0.07 |
| **Race** | | | | | | 0.033 |
| | White | 15 | 83% | 19 | 58% | |
| | Black | 1 | 6% | 4 | 12% | |
| | Other | 2 | 11% | 10 | 30% | |
| | **PSA** | 6.8 | | 5.7 | | 0.39 |
| **Clinical Gleason** | | | | | | |
| | <7 | - | | 15 | 38% | |
| | 7 | - | | 18 | 46% | |
| | >7 | - | | 6 | 16% | |
| **Clinical Stage** | | | | | | |
| | T1c | - | | 34 | 87% | |
| | >T1c | - | | 5 | 13% | |
| **Number of Cores** | | 15 | | 12 | | 0.006 |

As shown in Figure 20, the AUC value for the combination of ERG and AMACR was 0.8319 based on the expression of ERG and AMACR in 45 patients. The standard deviation was 0.0625. The 95% confidence Internal was between 0.70941 and 0.95439. Among the 45 patients, there were 16 biopsy negative patients and 29 biopsy positive patients. The information of age, race, clinical Gleason, clinical stage, and the number of biopsy cores for these 45 patients are shown in Table 11.

**Table 11. The 45 patients in the ROC analysis of the combination of ERG and AMACR**

| | | **Biopsy Neg** | | **Biopsy Pos** | | **p-value** |
|---|---|---|---|---|---|---|
| | **N** | 16 | | 29 | | |
| | **Age** | 68 | | 64 | | 0.02 |
| **Race** | | | | | | 0.033 |
| | White | 10 | 77% | 12 | 50% | |
| | Black | 1 | 8% | 3 | 13% | |
| | Other | 2 | 15% | 9 | 37% | |
| | **PSA** | 6.3 | | 5.9 | | 0.75 |
| **Clinical Gleason** | | | | | | |
| | <7 | - | | 12 | 41% | |
| | 7 | - | | 14 | 48% | |
| | >7 | - | | 3 | 10% | |
| **Clinical Stage** | | | | | | |
| | T1c | - | | 24 | 83% | |
| | >T1c | - | | 5 | 17% | |
| **Number of Cores** | | 14 | | 13 | | 0.25 |

The AUC value for the combination of the biomarkers ERG and AMACR was 0.8319. In contrast, the AUC value for ERG alone was 0.7824 and the AUC value for AMACR alone was 0.7610. The AUC value of 0.83 for the combination of ERG and AMACR was higher than the value for either ERG or AMACR alone, and therefore the combination of ERG and AMACR may be of higher diagnostic value than either ERG or AMACR alone.

In a second analysis, GAPDH was used as a reference gene, the expression of ERG and AMACR was measured, and ROC analysis was performed. The AUC value for a combination of the biomarkers ERG and alpha-methylacyl-CoA racemase (AMACR) was 0.88. In contrast, the AUC value for ERG alone was 0.70 and the AUC value for AMACR alone was 0.51. The AUC value of 0.88 for the combination of ERG and AMACR was higher than the value for either ERG or AMACR alone, and therefore may be of higher diagnostic value.

In a third analysis, KLK3 was used as a reference gene similar to the first analysis, the expression of PCA3 was measured, and ROC analysis was performed for PCA3 alone, and a combination of ERG and PCA3. As shown in Figure 18, the AUC value for ERG alone was 0.7824.

As shown in Figure 21, the AUC value for PCA3 alone was 0.6615 based on the PCA3 expression in urine samples from 116 patients. The standard deviation was 0.0512. The 95% confidence Internal was between 0.56107 and 0.76185. Among the 116 patients, there were 48 biopsy negative patients and 68 biopsy positive patients. The information of age, race, clinical Gleason, clinical stage, and the number of biopsy cores for these 116 patients are shown in Table 12.

**Table 12. The 116 patients in the ROC analysis of the combination of ERG and AMACR**

| | | **Biopsy Neg** | | **Biopsy Pos** | | **p-value** |
|---|---|---|---|---|---|---|
| | **N** | 48 | | 68 | | |
| | **Age** | 67 | | 66 | | 0.50 |
| **Race** | | | | | | 0.033 |
| | White | 33 | 80% | 35 | 56% | |
| | Black | 1 | 3% | 9 | 15% | |
| | Other | 7 | 17% | 18 | 29% | |
| | **PSA** | 6.0 | | 6.9 | | 0.40 |
| **Clinical Gleason** | | | | | | |
| | <7 | - | | 32 | 47% | |
| | 7 | - | | 27 | 40% | |
| | >7 | - | | 9 | 13% | |
| **Clinical Stage** | | | | | | |
| | T1c | - | | 61 | 90% | |
| | >T1c | - | | 7 | 10% | |
| **Number of Cores** | | 16 | | 14 | | 0.17 |

As shown in Figure 22, the AUC value for the combination of ERG and PCA3 was 0.7867 based on the expression of ERG and PCA3 in 93 patients. The 95% confidence Internal was between 0.69470 and 0.87866. Among the 93 patients, there were 37 biopsy negative patients and 56 biopsy positive patients. The information of age, race, clinical Gleason, clinical stage, and the number of biopsy cores for these 93 patients are shown in Table 13.

**Table 13. The 93 patients in the ROC analysis of the combination of ERG and AMACR**

| | | **Biopsy Neg** | | **Biopsy Pos** | | **p-value** |
|---|---|---|---|---|---|---|
| | **N** | 37 | | 56 | | |
| | **Age** | 68 | | 67 | | 0.48 |
| **Race** | | | | | | 0.033 |
| | White | 27 | 79% | 26 | 51% | |
| | Black | 1 | 3% | 8 | 16% | |
| | Other | 6 | 18% | 17 | 33% | |
| | **PSA** | 5.4 | | 6.6 | | 0.23 |
| **Clinical Gleason** | | | | | | |
| | <7 | - | | 27 | 48% | |
| | 7 | - | | 23 | 41% | |
| | >7 | - | | 6 | 11% | |
| **Clinical Stage** | | | | | | |
| | T1c | - | | 49 | 88% | |
| | >T1c | - | | 7 | 12% | |
| **Number of Cores** | | 15 | | 14 | | 0.63 |

The AUC value for the combination of the biomarkers ERG and PCA3 was 0.7867. In contrast, the AUC value for ERG alone was 0.7824 and the AUC value for PCA3 alone was 0.6615. The AUC value of 0.7867 for the combination of ERG and PCA3 was similar to the value for either ERG alone although it is higher than the value for PCA3 alone. Therefore the combination of ERG and PCA3 does not significantly improve the diagnostic value in comparison to ERG alone.

In addition to the ERG and AMACR combination, it is expected that some combinations of biomarkers as listed in Table 5 may have higher AUC values than each of the individual biomarkers in the combination. The combination may be a combination of biomarkers comprising any 2, 3, 4, 5, 6, 7, 8, 9, 10, 11, 12, 13, 14, 15, 16, 17, 18 or 19 of those biomarkers in Table 5. Routine experimentation by a person skilled in the art is able to identify which of the above combinations have higher AUC values and therefore have higher values for clinical applications such as disease diagnostics.

In addition to the ERG and PCA3 combination, it is expected that some combinations of biomarkers as listed in Table 5 may not have higher AUC values than each of the individual biomarkers in the combination. The combination may be a combination of biomarkers comprising any 2, 3, 4, 5, 6, 7, 8, 9, 10, 11, 12, 13, 14, 15, 16, 17, 18 or 19 of those biomarkers in Table 5. Routine experimentation by a person skilled in the art is able to identify which of the above combinations do not have higher AUC values and therefore do not have higher values for clinical applications such as disease diagnostics.

### References

Al-Nedawi, K., B. Meehan, J. Micallef, V. Lhotak, L. May, A. Guha, and J. Rak. 2008. Intercellular transfer of the oncogenic receptor EGFRvIII by microvesicles derived from tumour cells. Nat Cell Biol. 10:619-24.
Balzar, M., M.J. Winter, C.J. de Boer, and S.V. Litvinov. 1999. The biology of the 17-1A antigen (Ep-CAM). J Mol Med. 77:699-712.
Bossi, A., F. Bonini, A.P. Turner, and S.A. Piletsky. 2007. Molecularly imprinted polymers for the recognition of proteins: the state of the art. Biosens Bioelectron. 22:1131-7.
Bussemakers, M.J., A. van Bokhoven, G.W. Verhaegh, F.P. Smit, H.F. Karthaus, J.A. Schalken, F.M. Debruyne, N. Ru, and W.B. Isaacs. 1999. DD3: a new prostate-specific gene, highly overexpressed in prostate cancer. Cancer Res. 59:5975-9.
Chen, C., J. Skog, C.H. Hsu, R.T. Lessard, L. Balaj, T. Wurdinger, B.S. Carter, X.O. Breakefield, M. Toner, and D. Irimia. 2010. Microfluidic isolation and transcriptome analysis of serum microvesicles. Lab Chip. 10:505-11.
Cheruvanky, A., H. Zhou, T. Pisitkun, J.B. Kopp, M.A. Knepper, P.S. Yuen, and R.A. Star. 2007. Rapid isolation of urinary exosomal biomarkers using a nanomembrane ultrafiltration concentrator. Am J Physiol Renal Physiol. 292:F1657-61.
Cotton, R.G., N.R. Rodrigues, and R.D. Campbell. 1988. Reactivity of cytosine and thymine in single-base-pair mismatches with hydroxylamine and osmium tetroxide and its application to the study of mutations. Proc Natl Acad Sci USA. 85:4397-401.
Cowell, J.K., and K.C. Lo. 2009. Application of oligonucleotides arrays for coincident comparative genomic hybridization, ploidy status and loss of heterozygosity studies in human cancers. Methods Mol Biol. 556:47-65.
Deras, I.L., S.M. Aubin, A. Blase, J.R. Day, S. Koo, A.W. Partin, W.J. Ellis, L.S. Marks, Y. Fradet, H. Rittenhouse, and J. Groskopf. 2008. PCA3: a molecular urine assay for predicting prostate biopsy outcome. J Urol. 179:1587-92.
Furusato, B., C.L. Gao, L. Ravindranath, Y. Chen, J. Cullen, D.G. McLeod, A. Dobi, S. Srivastava, G. Petrovics, and I.A. Sesterhenn. 2008. Mapping of TMPRSS2-ERG fusions in the context of multi-focal prostate cancer. Mod Pathol. 21:67-75.
Guatelli, J.C., K.M. Whitfield, D.Y. Kwoh, K.J. Barringer, D.D. Richman, and T.R. Gingeras. 1990. Isothermal, in vitro amplification of nucleic acids by a multi enzyme reaction modeled after retroviral replication. Proc Natl Acad Sci USA. 87:1874-8.
Hahn, P.J. 1993. Molecular biology of double-minute chromosomes. Bioessays. 15:477-84.
Hessels, D., F.P. Smit, G.W. Verhaegh, J.A. Witjes, E.B. Cornel, and J.A. Schalken. 2007. Detection of TMPRSS2-ERG fusion transcripts and prostate cancer antigen 3 in urinary sediments may improve diagnosis of prostate cancer. Clin Cancer Res. 13:5103-8.
Johnson, S., D. Evans, S. Laurenson, D. Paul, A.G. Davies, P.K. Ferrigno, and C. Walti. 2008. Surface-immobilized peptide aptamers as probe molecules for protein detection. Anal Chem. 80:978-83.
Kwoh, D.Y., G.R. Davis, K.M. Whitfield, H.L. Chappelle, L.J. DiMichele, and T.R. Gingeras. 1989. Transcription-based amplification system and detection of amplified human immunodeficiency virus type 1 with a bead-based sandwich hybridization format. Proc Natl Acad Sci USA. 86:1173-7.
Laxman, B., D.S. Morris, J. Yu, J. Siddiqui, J. Cao, R. Mehra, R.J. Lonigro, A. Tsodikov, J.T. Wei, S.A. Tomlins, and A.M. Chinnaiyan. 2008. A first-generation multiplex biomarker analysis of urine for the early detection of prostate cancer. Cancer Res. 68:645-9.
Laxman, B., S.A. Tomlins, R. Mehra, D.S. Morris, L. Wang, B.E. Helgeson, R.B. Shah, M.A. Rubin, J.T. Wei, and A.M. Chinnaiyan. 2006. Noninvasive detection of TMPRSS2:ERG fusion transcripts in the urine of men with prostate cancer. Neoplasia. 8:885-8.
Li, J., L. Wang, H. Mamon, M.H. Kulke, R. Berbeco, and G.M. Makrigiorgos. 2008. Replacing PCR with COLD-PCR enriches variant DNA sequences and redefines the sensitivity of genetic testing. Nat Med. 14:579-84.
Lipson, D., T. Raz, A. Kieu, D.R. Jones, E. Giladi, E. Thayer, J.F. Thompson, S. Letovsky, P. Milos, and M. Causey. 2009. Quantification of the yeast transcriptome by single-molecule sequencing. Nat Biotechnol. 27:652-8.
Mattick, J.S. 2004. RNA regulation: a new genetics? Nat Rev Genet. 5:316-23.
Miele, E.A., D.R. Mills, and F.R. Kramer. 1983. Autocatalytic replication of a recombinant RNA J Mol Biol. 171:281-95.
Myers, R.M., Z. Larin, and T. Maniatis. 1985. Detection of single base substitutions by ribonuclease cleavage at mismatches in RNA:DNA duplexes. Science. 230:1242-6.
Nguyen, P.N., P. Violette, S. Chan, S. Tanguay, W. Kassouf, A. Aprikian, and J.Z. Chen. 2011. A panel of TMPRSS2:ERG fusion transcript markers for urine-based prostate cancer detection with high specificity and sensitivity. Eur Urol. 59:407-14.
Nilsson, J., J. Skog, A. Nordstrand, V. Baranov, L. Mincheva-Nilsson, X.O. Breakefield, and A. Widmark. 2009. Prostate cancer-derived urine exosomes: a novel approach to biomarkers for prostate cancer. Br J Cancer. 100:1603-7.
Orita, M., H. Iwahana, H. Kanazawa, K. Hayashi, and T. Sekiya. 1989. Detection of polymorphisms of human DNA by gel electrophoresis as single-strand conformation polymorphisms. Proc Natl Acad Sci USA. 86:2766-70.
Orozco, A.F., and D.E. Lewis. 2010. Flow cytometric analysis of circulating microparticles in plasma. Cytometry A. 77:502-14.
Palanisamy, N., B. Ateeq, S. Kalyana-Sundaram, D. Pflueger, K. Ramnarayanan, S. Shankar, B. Han, Q. Cao, X. Cao, K. Suleman, C. Kumar-Sinha, S.M. Dhanasekaran, Y.B. Chen, R. Esgueva, S. Banerjee, C.J. LaFargue, J. Siddiqui, F. Demichelis, P. Moeller, T.A. Bismar, R. Kuefer, D.R. Fullen, T.M. Johnson, J.K. Greenson, T.J. Giordano, P. Tan, S.A. Tomlins, S. Varambally, M.A. Rubin, C.A. Maher, and A.M. Chinnaiyan. 2010. Rearrangements of the RAF kinase pathway in prostate cancer, gastric cancer and melanoma. Nat Med. 16:793-8.
Petrovics, G., A. Liu, S. Shaheduzzaman, B. Furusato, C. Sun, Y. Chen, M. Nau, L. Ravindranath, A. Dobi, V. Srikantan, I.A. Sesterhenn, D.G. McLeod, M. Vahey, J.W. Moul, and S. Srivastava. 2005. Frequent overexpression of ETS-related gene-1 (ERG1) in prostate cancer transcriptome. Oncogene. 24:3847-52.
Raposo, G., H.W. Nijman, W. Stoorvogel, R. Liejendekker, C.V. Harding, C.J. Melief, and H.J. Geuze. 1996. B lymphocytes secrete antigen-presenting vesicles. J Exp Med. 183:1161-72.
Rice, K.R., Y. Chen, A. Ali, E.J. Whitman, A. Blase, M. Ibrahim, S. Elsamanoudi, S. Brassell, B. Furusato, N. Stingle, I.A. Sesterhenn, G. Petrovics, S. Miick, H. Rittenhouse, J. Groskopf, D.G. McLeod, and S. Srivastava. 2010. Evaluation of the ETS-related gene mRNA in urine for the detection of prostate cancer. Clin Cancer Res. 16:1572-6.
Rostad, K., O.J. Hellwinkel, S.A. Haukaas, O.J. Halvorsen, A.M. Oyan, A. Haese, L. Budaus, H. Albrecht, L.A. Akslen, T. Schlomm, and K.H. Kalland. 2009. TMPRSS2:ERG fusion transcripts in urine from prostate cancer patients correlate with a less favorable prognosis. APMIS. 117:575-82.
Salami, S.S., F. Schmidt, B. Laxman, M.M. Regan, D.S. Rickman, D. Scherr, G. Bueti, J. Siddiqui, S.A. Tomlins, J.T. Wei, A.M. Chinnaiyan, M.A. Rubin, and M.G. Sanda. 2011. Combining urinary detection of TMPRSS2:ERG and PCA3 with serum PSA to predict diagnosis of prostate cancer. Urol Oncol.
Skog, J., T. Wurdinger, S. van Rijn, D.H. Meijer, L. Gainche, M. Sena-Esteves, W.T. Curry, Jr., B.S. Carter, A.M. Krichevsky, and X.O. Breakefield. 2008. Glioblastoma microvesicles transport RNA and proteins that promote tumour growth and provide diagnostic biomarkers. Nat Cell Biol. 10:1470-6.
Steemers, F.J., W. Chang, G. Lee, D.L. Barker, R. Shen, and K.L. Gunderson. 2006. Whole-genome genotyping with the single-base extension assay. Nat Methods. 3:31-3.
Taylor, D.D., and C. Gercel-Taylor. 2008. MicroRNA signatures of tumor-derived exosomes as diagnostic biomarkers of ovarian cancer. Gynecol Oncol. 110:13-21.
Ting, D.T., D. Lipson, S. Paul, B.W. Brannigan, S. Akhavanfard, E.J. Coffman, G. Contino, V. Deshpande, A.J. Iafrate, S. Letovsky, M.N. Rivera, N. Bardeesy, S. Maheswaran, and D.A. Haber. 2011. Aberrant overexpression of satellite repeats in pancreatic and other epithelial cancers. Science. 331:593-6.
Tomlins, S.A., S.M. Aubin, J. Siddiqui, R.J. Lonigro, L. Sefton-Miller, S. Miick, S. Williamsen, P. Hodge, J. Meinke, A. Blase, Y. Penabella, J.R. Day, R. Varambally, B. Han, D. Wood, L. Wang, M.G. Sanda, M.A. Rubin, D.R. Rhodes, B. Hollenbeck, K. Sakamoto, J.L. Silberstein, Y. Fradet, J.B. Amberson, S. Meyers, N. Palanisamy, H. Rittenhouse, J.T. Wei, J. Groskopf, and A.M. Chinnaiyan. 2011. Urine TMPRSS2:ERG Fusion Transcript Stratifies Prostate Cancer Risk in Men with Elevated Serum PSA. Sci Transl Med. 3:94ra72.
Tomlins, S.A., D.R. Rhodes, S. Perner, S.M. Dhanasekaran, R. Mehra, X.W. Sun, S. Varambally, X. Cao, J. Tchinda, R. Kuefer, C. Lee, J.E. Montie, R.B. Shah, K.J. Pienta, M.A. Rubin, and A.M. Chinnaiyan. 2005. Recurrent fusion of TMPRSS2 and ETS transcription factor genes in prostate cancer. Science. 310:644-8.
Velculescu, V.E., L. Zhang, B. Vogelstein, and K.W. Kinzler. 1995. Serial analysis of gene expression. Science. 270:484-7.
Went, P.T., A. Lugli, S. Meier, M. Bundi, M. Mirlacher, G. Sauter, and S. Dirnhofer. 2004. Frequent EpCam protein expression in human carcinomas. Hum Pathol. 35:122-8.
Wong, M.L., and J.F. Medrano. 2005. Real-time PCR for mRNA quantitation. Biotechniques. 39:75-85.
Zhang, M., D.E. Latham, M.A. Delaney, and A. Chakravarti. 2005. Survivin mediates resistance to antiandrogen therapy in prostate cancer. Oncogene. 24:2474-82.

## Claims

1. A method for diagnosis or monitoring of prostate cancer in a subject, comprising the steps of:
(a) obtaining a microvesicle fraction from a urine sample from a subject, where the sample is collected without the use of a digital rectal exam (DRE) or prostatic massage prior to urine collection;
(b) extracting one or more nucleic acids from the microvesicle fraction; and
(c) analyzing the extracted nucleic acids to detect the level of expression of two biomarkers associated with prostate cancer, wherein the first biomarker is one or more isoforms of ERG and the second biomarker is PCA3.

2. A method according to claim 1, wherein step (a) comprises processing the urine sample to remove cells and cell debris while retaining a microvesicle fraction from the urine sample and wherein step (c) comprises (d) detecting a level of expression for two biomarkers associated with prostate cancer in the extracted nucleic acids, wherein the first biomarker is one or more isoforms of ERG and the second biomarker is PCA3, and detecting a level of expression of a reference gene; and (e) determining a normalized, relative expression level of the ERG and PCA3, wherein the relative expression level of the ERG or PCA3 is a ratio between the level of ERG or PCA3 expression to the level of reference gene expression; and
wherein the subject is identified as suffering from, or being at an increased risk for prostate cancer when the relative expression level of the ERG and PCA3 is greater than a cutoff level of expression.

3. The method of claim 2, wherein the cutoff level of expression is a score based on a collective level of ERG and PCA3 expression in a control group of subjects that are not suffering from the medical condition of the prostate or a score based on a collective level of ERG and PCA3 expression in a control group of subjects that have been diagnosed with a low level or early stage of the medical condition of the prostate gland.

4. The method of claim 1 or claim 2, wherein the one or more isoforms of ERG are one or more of ERG1, ERG2, ERG3, ERG4, ERG5, ERG6, ERG7, ERG8, or ERG9.

5. The method of claim 1 or claim 2, wherein the biomarker is RNA.

6. The method of claim 1 or claim 2, wherein the biomarker is an RNA expression profile.

7. The method of claim 6, wherein the RNA expression profile is:
a combination of an RNA expression profile of one or more isoforms of the ERG gene, and an RNA expression profile of PCA3; or
an RNA expression profile of one or more isoforms of ERG and PCA3 in combination with an RNA expression profile of one or more isoforms of a gene selected from the group consisting of, TMPRSS2-ERG, Survivin, AMACR, AKT1, AMD1, ANXA3, EEF2, EZH2, GSTP1, HFM1, MMP9, MSMB, NCOA2, , PMEPA1, PSCA, PSGR, RAD21, SMAD4, TGM4, and KLK3.

8. The method of claim 1 or claim 2, wherein the subject has previously undergone a prostate biopsy.

9. The method of claim 2, wherein the reference gene is a prostate-specific gene, GAPDH, KLK3 or a combination thereof.

10. The method of claim 2, wherein said processing the urine sample to remove cells and cell debris while retaining microvesicle fraction comprises a step of filtration concentration.

11. The method of claim 10, wherein the filtration concentration step uses a filter having a molecular weight cutoff that retains the microvesicle fraction and removes all other cell fractions and cell debris.

12. The method of claim 11, wherein the filter has a molecular weight cutoff of at least 100 kDa.

## Patentansprüche

1. Verfahren zur Diagnose oder Überwachung von Prostatakrebs in einem Subjekt, umfassend die folgenden Schritte:
(a) Gewinnen einer Mikrovesikelfraktion aus einer Urinprobe eines Subjekts, wobei die Probe ohne die Verwendung einer digitalen rektalen Untersuchung (DRE) oder einer Prostatamassage vor der Urinsammlung gesammelt wird;
(b) Extrahieren einer oder mehrerer Nukleinsäuren aus der Mikrovesikelfraktion; und
(c) Analysieren der extrahierten Nukleinsäuren zum Nachweis des Expressionsniveaus von zwei Biomarkern, die mit Prostatakrebs assoziiert sind, wobei der erste Biomarker eine oder mehrere Isoformen von ERG ist und der zweite Biomarker PCA3 ist.

2. Verfahren nach Anspruch 1, wobei Schritt (a) die Verarbeitung der Urinprobe umfasst, um Zellen und Zelltrümmer zu entfernen, während eine Mikrovesikelfraktion aus der Urinprobe zurückbehalten wird, und wobei Schritt (c) umfasst: (d) Nachweisen eines Expressionsniveaus für zwei mit Prostatakrebs assoziierte Biomarker in den extrahierten Nukleinsäuren, wobei der erste Biomarker eine oder mehrere Isoformen von ERG ist und der zweite Biomarker PCA3 ist, und Nachweisen eines Expressionsniveaus eines Referenzgens; und (e) Bestimmen eines normalisierten, relativen Expressionsniveaus von ERG und PCA3, wobei das relative Expressionsniveau von ERG oder PCA3 ein Verhältnis zwischen dem Niveau der ERG- oder PCA3-Expression und dem Niveau der Referenzgenexpression ist; und
wobei das Subjekt als an Prostatakrebs leidend oder mit einem erhöhten Risiko für Prostatakrebs behaftet identifiziert wird, wenn das relative Expressionsniveau von ERG und PCA3 größer ist als eine Expressions-Ausschlussgrenze.

3. Verfahren nach Anspruch 2, wobei die Expressions-Ausschlussgrenze ein Score auf Basis eines Kollektivniveaus der ERG- und PCA3-Expression in einer Kontrollgruppe von Subjekten ist, die nicht an der Erkrankung der Prostata leiden, oder ein Score auf Basis eines Kollektivniveaus der ERG- und PCA3-Expression in einer Kontrollgruppe von Subjekten, bei denen ein niedriges Niveau oder ein frühes Stadium des medizinischen Zustands der Prostata diagnostiziert wurde.

4. Verfahren nach Anspruch 1 oder Anspruch 2, wobei die eine oder mehrere Isoformen von ERG eine oder mehrere von ERG1, ERG2, ERG3, ERG4, ERG5, ERG6, ERG7, ERG8 oder ERG9 sind.

5. Verfahren nach Anspruch 1 oder Anspruch 2, wobei der Biomarker RNA ist.

6. Verfahren nach Anspruch 1 oder Anspruch 2, wobei der Biomarker ein RNA-Expressionsprofil ist.

7. Verfahren nach Anspruch 6, wobei das RNA-Expressionsprofil ist:
eine Kombination aus einem RNA-Expressionsprofil einer oder mehrerer Isoformen des ERG-Gens und einem RNA-Expressionsprofil von PCA3; oder
ein RNA-Expressionsprofil einer oder mehrerer Isoformen von ERG und PCA3 in Kombination mit einem RNA-Expressionsprofil einer oder mehrerer Isoformen eines Gens, ausgewählt aus der Gruppe bestehend aus TMPRSS2-ERG, Survivin, AMACR, AKT1, AMD1, ANXA3, EEF2, EZH2, GSTP1, HFM1, MMP9, MSMB, NCOA2, PMEPA1, PSCA, PSGR, RAD21, SMAD4, TGM4, und KLK3.

8. Verfahren nach Anspruch 1 oder Anspruch 2, wobei das Subjekt zuvor einer Prostatabiopsie unterzogen wurde.

9. Verfahren nach Anspruch 2, wobei das Referenzgen ein prostataspezifisches Gen, GAPDH, KLK3 oder eine Kombination davon ist.

10. Verfahren nach Anspruch 2, wobei die Verarbeitung der Urinprobe zur Entfernung von Zellen und Zelltrümmern unter Beibehaltung der Mikrovesikelfraktion einen Schritt der Filtrationskonzentrierung umfasst.

11. Verfahren nach Anspruch 10, wobei der Filtrationskonzentrierungsschritt einen Filter mit einer Molekulargewichts-Ausschlussgrenze verwendet, der die Mikrovesikelfraktion zurückhält und alle anderen Zellfraktionen und Zelltrümmer entfernt.

12. Verfahren nach Anspruch 11, wobei der Filter eine Molekulargewichts-Ausschlussgrenze von mindestens 100 kDa aufweist.

## Revendications

1. Procédé destiné à diagnostiquer ou à suivre un cancer de la prostate chez un sujet, comprenant les étapes :
(a) d'obtention d'une fraction micro-vésiculaire à partir d'un échantillon d'urine qui provient d'un sujet, où l'échantillon est prélevé sans utiliser un toucher rectal (TR) ou un massage prostatique préalable au recueil de l'urine ;
(b) d'extraction d'un ou de plusieurs acide(s) nucléique(s) de la fraction micro-vésiculaire ; et
(c) d'analyser les acides nucléiques extraits pour détecter le niveau d'expression de deux biomarqueurs associés à un cancer de la prostate, dans lequel le premier biomarqueur est une ou sont plusieurs isoforme(s) de l'ERG et le deuxième biomarqueur est le PCA3.

2. Procédé selon la revendication 1, dans lequel l'étape (a) comprend un traitement de l'échantillon d'urine pour ôter les cellules et débris cellulaires tout en retenant une fraction micro-vésiculaire à partir de l'échantillon d'urine, et dans lequel l'étape (c) comprend (d) une détection d'un niveau d'expression pour deux biomarqueurs associés à un cancer de la prostate dans les acides nucléiques extraits, dans lequel le premier biomarqueur est une ou sont plusieurs isoforme(s) de l'ERG et le deuxième biomarqueur est le PCA3, et une détection d'un niveau d'expression d'un gène de référence ; et (e) une détermination d'un niveau d'expression relative, normalisé de l'ERG et du PCA3, dans lequel le niveau d'expression relative de l'ERG ou du PCA3 est un rapport entre le niveau de l'expression de l'ERG ou du PCA3 et le niveau de l'expression du gène de référence ; et
dans lequel le sujet est identifié comme souffrant d'un cancer de la prostate ou présentant un risque accru de celui-ci quand le niveau d'expression relative de l'ERG et du PCA3 est supérieur à un niveau seuil de l'expression.

3. Procédé de la revendication 2, dans lequel le niveau seuil de l'expression est un score basé sur un niveau collectif de l'expression de l'ERG et du PAC3 dans un groupe témoin de sujets qui ne souffrent pas de la condition médicale de la prostate ou bien un score basé sur un niveau collectif de l'expression de l'ERG et du PAC3 dans un groupe témoin de sujets qui ont été diagnostiqués comme ayant un faible niveau de la condition médicale de la glande prostatique ou étant à un stade précoce de celle-ci.

4. Procédé de la revendication 1 ou la revendication 2, dans lequel l'une ou les plusieurs isoforme(s) de l'ERG est/sont une ou plusieurs parmi les ERG1, ERG2, ERG3, ERG4, ERGS, ERG6, ERG7, ERG8 ou ERG9.

5. Procédé de la revendication 1 ou la revendication 2, dans lequel le biomarqueur est l'ARN.

6. Procédé de la revendication 1 ou la revendication 2, dans lequel le biomarqueur est un profil d'expression de l'ARN.

7. Procédé de la revendication 6, dans lequel le profil d'expression de l'ARN est :
une combinaison d'un profil d'expression de l'ARN d'une ou de plusieurs isoforme (s) du gène de l'ERG et d'un profil d'expression de l'ARN du PCA3 ; ou
un profil d'expression de l'ARN d'une ou de plusieurs isoforme(s) de l'ERG et du PCA3 en combinaison avec un profil d'expression de l'ARN d'une ou de plusieurs isoforme (s) d'un gène choisi dans le groupe constitué par les TMPRSS2-ERG, Survivine, AMACR, AKT1, AMD1, ANXA3, EEF2, EZH2, GSTP1, HFM1, MMP9, MSMB, NCOA2, PMEPA1, PSCA, PSGR, RAD21, SMAD4, TGM4 et KLK3.

8. Procédé de la revendication 1 ou la revendication 2, dans lequel le sujet a préalablement subi une biopsie de la prostate.

9. Procédé de la revendication 2, dans lequel le gène de référence est un gène spécifique de la prostate, le GAPDH, le KLK3 ou une combinaison de ceux-ci.

10. Procédé de la revendication 2, dans lequel ledit traitement de l'échantillon d'urine, dans le but d'ôter les cellules et débris cellulaires tout en retenant la fraction micro-vésiculaire, comprend une étape de concentration par filtration.

11. Procédé de la revendication 10, dans lequel l'étape de concentration par filtration utilise un filtre ayant un seuil de poids moléculaire qui retient la fraction micro-vésiculaire et ôte l'ensemble des autres fractions cellulaires et débris cellulaires.

12. Procédé de la revendication 11, dans lequel le filtre a un seuil de poids moléculaire d'au moins 100 kDa.
